# EUROPEAN PATENT APPLICATION

(11) **EP 2 065 475 A1**
(43) Date of publication of application: **03.06.2009**
(21) Application number: 07121983.6
(22) Date of filing: 30.11.2007
(51) Int. Cl.: C12Q 1/68

(54) **Method for therapy prediction in tumors having irregularities in the expression of at least one VEGF ligand and/or at least one ErbB-receptor**

(71) Applicant: Siemens Healthcare Diagnostics GmbH, 65760 Eschborn (DE)
(72) Inventor: Fountzilas, George, 55236 Panorama, Thessaloniki (GR); Hennig, Guido, 50859 Köln (DE); Stropp, Udo, 42781 Haan (DE); Wirtz, Ralph, 50677 Köln (DE)
(74) Representative: Maier, Daniel Oliver

(57) **Abstract**

The present invention is related to a method for predicting a clinical response of a patient suffering from or at risk of developing a neoplastic disease towards a given mode of treatment, said method comprising the steps of:
a) obtaining a biological sample from said patient;
b) determining, on a non protein basis, the expression level of at least one gene encoding for a ligand from the Vascular endothelial growth factor (VEGF) family and of and of at least one gene encoding for a receptor from the ErbB-family, or a gene co-expressed therewith, in said sample,
c) comparing the pattern of expression levels determined in (b) with one or several reference pattern(s) of expression levels; and
d) predicting therapeutic success for said given mode of treatment in said patient or implementing therapeutic regimen targeting the signalling pathway of said ligand and/or receptor is related to in said patient from the outcome of the comparison in step (c).

SEQUENCE LISTING

<110>  Siemens AG

<120>  A method for predicting a clinical response of a patient
       suffering from or at risk of developing a neoplastic disease
       towards a given mode of treatment

<130>  SD40183

<160>  66

<170>  PatentIn version 3.3

<210>  1
<211>  28
<212>  DNA
<213>  Artificial

<220>
<223>  VEGFA

<400>  1
cacattgttg gaagaagcag cccatgac                                          28


<210>  2
<211>  18
<212>  DNA
<213>  artificial

<220>
<223>  VEGFA

<400>  2
cagatgtccc ggcgaaga                                                     18


<210>  3
<211>  18
<212>  DNA
<213>  artificial

<220>
<223>  VEGFA

<400>  3
gagggcgagt cccaggaa                                                     18


<210>  4
<211>  25
<212>  DNA
<213>  artificial

<220>
<223>  VEGFA

<400>  4
cgttcgttta actcaagctg cctcg                                             25


<210>  5
<211>  20
<212>  DNA
<213>  artificial

<220>

<223>  VEGFA

<400>  5
aacacagact cgcgttgcaa                                                    20


<210>  6
<211>  21
<212>  DNA
<213>  artificial

<220>
<223>  VEGFA

<400>  6
cggcttgtca catctgcaag t                                                  21


<210>  7
<211>  31
<212>  DNA
<213>  artificial

<220>
<223>  VEGFA

<400>  7
aacttcctcg ggttcataac catagcagtc c                                       31


<210>  8
<211>  22
<212>  DNA
<213>  artificial

<220>
<223>  VEGFA

<400>  8
cccccaacat ctggttagtc tt                                                 22


<210>  9
<211>  19
<212>  DNA
<213>  artificial

<220>
<223>  VEGFA

<400>  9
ccacgggcac agaatatgc                                                     19


<210>  10
<211>  28
<212>  DNA
<213>  artificial

<220>
<223>  VEGFA

<400>  10
caccatgcag attatgcgga tcaaacct                                           28


<210>  11
<211>  20

<212>  DNA
<213>  artificial

<220>
<223>  VEGFA

<400>  11
gcccactgag gagtccaaca                                                    20


<210>  12
<211>  20
<212>  DNA
<213>  artificial

<220>
<223>  VEGFA

<400>  12
tcctatgtgc tggccttggt                                                    20


<210>  13
<211>  28
<212>  DNA
<213>  artificial

<220>
<223>  VEGFA

<400>  13
caccatgcag attatgcgga tcaaacct                                           28


<210>  14
<211>  20
<212>  DNA
<213>  artificial

<220>
<223>  VEGFA

<400>  14
gcccactgag gagtccaaca                                                    20


<210>  15
<211>  20
<212>  DNA
<213>  artificial

<220>
<223>  VEGFA

<400>  15
gcctcggctt gtcacatttt                                                    20


<210>  16
<211>  28
<212>  DNA
<213>  artificial

<220>
<223>  VEGFA

<400>  16
caccatgcag attatgcgga tcaaacct                                           28

<210>  17
<211>  20
<212>  DNA
<213>  artificial

<220>
<223>  VEGFA

<400>  17
gcccactgag gagtccaaca                                                    20


<210>  18
<211>  20
<212>  DNA
<213>  artificial

<220>
<223>  VEGFA

<400>  18
agcaaggccc acagggattt                                                    20


<210>  19
<211>  28
<212>  DNA
<213>  artificial

<220>
<223>  VEGFA

<400>  19
caccatgcag attatgcgga tcaaacct                                           28


<210>  20
<211>  20
<212>  DNA
<213>  artificial

<220>
<223>  VEGFA

<400>  20
gcccactgag gagtccaaca                                                    20


<210>  21
<211>  21
<212>  DNA
<213>  artificial

<220>
<223>  VEGFA

<400>  21
aacgctccag gacttatacc g                                                  21


<210>  22
<211>  21
<212>  DNA
<213>  artificial

<220>

<223>  VEGFB

<400>  22
acagggctgc cactccccac c                                                21


<210>  23
<211>  26
<212>  DNA
<213>  artificial

<220>
<223>  VEGFB

<400>  23
aatgcagacc taaaaaaaag gacagt                                           26


<210>  24
<211>  17
<212>  DNA
<213>  artificial

<220>
<223>  VEGFB

<400>  24
cccagcccgg aacagaa                                                     17


<210>  25
<211>  31
<212>  DNA
<213>  artificial

<220>
<223>  VEGFB

<400>  25
cacatctatc catgacacca ctttcctctg g                                     31


<210>  26
<211>  19
<212>  DNA
<213>  artificial

<220>
<223>  VEGFB

<400>  26
tggcaggtag cgcgagtat                                                   19


<210>  27
<211>  19
<212>  DNA
<213>  artificial

<220>
<223>  VEGFB

<400>  27
ccctgtctcc cagcctgat                                                   19


<210>  28
<211>  30

<212>  DNA
<213>  artificial

<220>
<223>  VEGFB

<400>  28
ttcctcccct cactaagaag acccaaacct                                        30


<210>  29
<211>  24
<212>  DNA
<213>  artificial

<220>
<223>  VEGFB

<400>  29
ccactctgtg caagtaagca tctt                                              24


<210>  30
<211>  22
<212>  DNA
<213>  artificial

<220>
<223>  VEGFB

<400>  30
gtaccaaagc ccaaatccca tt                                                22


<210>  31
<211>  39
<212>  DNA
<213>  artificial

<220>
<223>  VEGFD

<400>  31
tgacattgaa acactaaaag ttatagatga agaatggca                              39


<210>  32
<211>  22
<212>  DNA
<213>  artificial

<220>
<223>  VEGFD

<400>  32
actaggtttg cggcaacttt ct                                                22


<210>  33
<211>  23
<212>  DNA
<213>  artificial

<220>
<223>  VEGFD

<400>  33
tctctagggc tgcactgagt tct                                               23

<210>  34
<211>  21
<212>  DNA
<213>  artificial

<220>
<223>  VEGFC

<400>  34
acggccatgt acgaaccgcc a                                                21


<210>  35
<211>  20
<212>  DNA
<213>  artificial

<220>
<223>  VEGFC

<400>  35
gttccaccac caaacatgca                                                  20


<210>  36
<211>  25
<212>  DNA
<213>  artificial

<220>
<223>  VEGFC

<400>  36
cactatatga aaatcctggc tcaca                                            25


<210>  37
<211>  21
<212>  DNA
<213>  artificial

<220>
<223>  VEGFC

<400>  37
aaacatggcc cggcgtcaac c                                                21


<210>  38
<211>  25
<212>  DNA
<213>  artificial

<220>
<223>  VEGFC

<400>  38
ccagaataga agtcatgctt tgatg                                            25


<210>  39
<211>  25
<212>  DNA
<213>  artificial

<220>

<223>  VEGFC

<400>  39
tttagatcag agcaaatgtc ttgca                                          25


<210>  40
<211>  27
<212>  DNA
<213>  artificial

<220>
<223>  VEGFC

<400>  40
ttgagtcatc tccagcatcc gaggaaa                                        27


<210>  41
<211>  22
<212>  DNA
<213>  artificial

<220>
<223>  VEGFC

<400>  41
ccacagatgt catggaatcc at                                             22


<210>  42
<211>  20
<212>  DNA
<213>  artificial

<220>
<223>  VEGFC

<400>  42
tgcctggctc aggaagattt                                                20


<210>  43
<211>  29
<212>  DNA
<213>  artificial

<220>
<223>  VEGFC

<400>  43
agaacaggcc aacctcaact caaggacag                                      29


<210>  44
<211>  21
<212>  DNA
<213>  artificial

<220>
<223>  VEGFC

<400>  44
gagatcccca tggaggtctt c                                              21


<210>  45
<211>  32

<212>  DNA
<213>  artificial

<220>
<223>  VEGFC

<400>  45
cactcattat caatactttt caagatctct gt                                    32


<210>  46
<211>  26
<212>  DNA
<213>  artificial

<220>
<223>  VEGFC

<400>  46
tgcatgccac gggaggtgtg tataga                                           26


<210>  47
<211>  24
<212>  DNA
<213>  artificial

<220>
<223>  VEGFC

<400>  47
aatagaccct ggagtgaaac catt                                             24


<210>  48
<211>  20
<212>  DNA
<213>  artificial

<220>
<223>  VEGFC

<400>  48
tattgcagca acccccacat                                                  20


<210>  49
<211>  27
<212>  DNA
<213>  artificial

<220>
<223>  VEGFC

<400>  49
acatgcagct gttacagacg gccatgt                                          27


<210>  50
<211>  21
<212>  DNA
<213>  artificial

<220>
<223>  VEGFC

<400>  50
ctgagcaagc ggtctctgag t                                                21

<210> 51
<211> 25
<212> DNA
<213> artificial

<220>
<223> VEGFC

<400> 51
cactatatga aaatcctggc tcaca 25


<210> 52
<211> 37
<212> DNA
<213> artificial

<220>
<223> VEGF-D

<400> 52
ccatcctcta ccagaacata catcagttat ttggaga 37


<210> 53
<211> 20
<212> DNA
<213> artificial

<220>
<223> VEGF-D

<400> 53
cccttcccac caagtgttca 20


<210> 54
<211> 19
<212> DNA
<213> artificial

<220>
<223> VEGF-D

<400> 54
tggtgctgcc tcactggat 19


<210> 55
<211> 22
<212> DNA
<213> artificial

<220>
<223> ERBB2

<400> 55
aggccaagtc cgcagaagcc ct 22


<210> 56
<211> 20
<212> DNA
<213> artificial

<220>

<223>  ERBB2

<400>  56
tctggacgtg ccagtgtgaa                                                    20


<210>  57
<211>  20
<212>  DNA
<213>  artificial

<220>
<223>  ERBB2

<400>  57
cctgctccct gaggacacat                                                    20


<210>  58
<211>  21
<212>  DNA
<213>  artificial

<220>
<223>  ERBB2

<400>  58
accaggaccc accagagcgg g                                                  21


<210>  59
<211>  22
<212>  DNA
<213>  artificial

<220>
<223>  ERBB2

<400>  59
ccagccttcg acaacctcta tt                                                 22


<210>  60
<211>  19
<212>  DNA
<213>  artificial

<220>
<223>  ERBB2

<400>  60
tgccgtaggt gtccctttg                                                     19


<210>  61
<211>  31
<212>  DNA
<213>  artificial

<220>
<223>  ERBB2

<400>  61
tgatcatggt caaatgttgg atgattgact c                                       31


<210>  62
<211>  23

<212>  DNA
<213>  artificial

<220>
<223>  ERBB2

<400>  62
ccatctgcac cattgatgtc tac                                              23


<210>  63
<211>  19
<212>  DNA
<213>  artificial

<220>
<223>  ERBB2

<400>  63
cggaatcttg gccgacatt                                                   19


<210>  64
<211>  22
<212>  DNA
<213>  artificial

<220>
<223>  ERBB2

<400>  64
aagattcccc ttcttcctgg ga                                               22


<210>  65
<211>  20
<212>  DNA
<213>  artificial

<220>
<223>  ERBB2

<400>  65
acgccctcag aagattggaa                                                  20


<210>  66
<211>  21
<212>  DNA
<213>  artificial

<220>
<223>  ERBB2

<400>  66
tgtgctgacg caagctacaa c                                                21

## Description

### Field of the invention

The present invention relates to methods for prediction of the therapeutic success of cancer therapy.

### Background of the invention

Disease free survival and overall survival of high risk breast cancer patients as determined by conventional clinical parameters (nodal Status, grade, tumor size) is critical with 20% tumor recurrence despite intensive treatment combining chemo- and endocrine therapy. However, molecular tests that better select a more appropriate therapy, e.g. by adding targeted anti-cancer drugs, are not available.

It has been shown from several studies that receptors from the ErbB-family (also termed "epidermal growth factor receptor" (EGFR) family) play an improtant role in cancer genesis. Said family comprises cell-surface receptors for, among others, members of the epidermal growth factor family (EGF-family) of extracellular protein ligands. The ErbB family of receptors is a family of four closely related receptor tyrosine kinases: EGFR (ErbB-1), HER-2/neu (ErbB-2), Her 3 (ErbB-3) and Her 4 (ErbB-4). It has been reported that mutations affecting ErbB expression or activity often result in cancer.

ErbB receptors are transmembrane protein receptors which are activated by binding of their specific ligands, including epidermal growth factor and transforming growth factor α (TGFα). Upon activation by its growth factor ligands, many ErbB receptors undergo a transition from an inactive monomeric form to an active homodimer or heterodimer, which then stimuates cell growth, tissue proliferation and cell mitosis, the mechanism of which will be described in the following.

The said ErbB comprises a tyrsoine kinase on its intracellular domain. ErbB dimerization stimulates the activity of said tyrosine kinase. As a result, autophosphorylation of five tyrosine (Y) residues in the C-terminal domain of ErbB occurs. These are Y992, Y1045, Y1068, Y1148 and Y1173. This autophosphorylation elicits downstream activation and signaling by several other proteins that associate with the phosphorylated tyrosines through their own phosphotyrosine-binding SH2 domains. These downstream signaling proteins initiate several signal transduction cascades, principally the MAPK, Akt and JNK pathways, leading to DNA synthesis and cell proliferation¹.

Such proteins modulate phenotypes such as cell migration, adhesion, and proliferation. The kinase domain of EGFR can also cross-phosphorylate tyrosine residues of other receptors it is aggregated with, and can itself be activated in that manner.

There is some evidence that in some cases preformed inactive dimers may also exist before ligand binding. In addition to forming homodimers after ligand binding, different members of the ErbB receptor family may pair with one another member such as ErbB2/Her-2/neu and EGFR-1, to create an activated heterodimer. Moreover, there is evidence that in some cancerogenic cells the overexpression of EGFR leads to an elevated abundance of said receptor in the cellular membranes, which leads to autonomous dimerization due to high receptor density, without the need for the ligand to elicit said dimerization.

Hence, an overexpression of either native or mutant receptors from the ErbB-family, like ErbB2/Her-2/neu, is frequently found in cancerogenic and pre-cancerogenic cells and/or tissues. Said overexpression may be accompanied by mutations of the EGFR gene itself, as well as to gene amplification, polysomy, aneuploidy, genomic instability, irregularities in the gene regulation, and the like. Said overexpression leads to a self-activation of cell proliferation in the respective cells and/or tissues due to autonomous dimerization. Overexpression of EGFR does thus trigger a positive feedback mechanism which rapidly enforces tumor growth.

Recently, Trastuzumab (trade name: Herceptin), a humanized monoclonal antibody which binds to the extracellular segment of the ErbB2 receptor, has been introduced as anti-cancer therapy in breast cancer.
Cells treated with trastuzumab undergo arrest during the G1 phase of the cell cycle so there is reduced proliferation. It has been suggested that trastuzumab induces some of its effect by downregulation of ErbB2 leading to disruption of receptor dimerization and signaling through the downstream PI3K cascade. P27Kip1 is then not phosphorylated and is able to enter the nucleus and inhibit cdk2 activity, causing cell cycle arrest. Also, trastuzumab suppresses angiogenesis by both induction of antiangiogenic factors and repression of proangiogenic factors. It is thought that a contribution to the unregulated growth observed in cancer could be due to proteolytic cleavage of ErbB2 that results in the release of the extracellular domain. Trastuzumab has been shown to inhibit erbB2 ectodomain cleavage in breast cancer cells. There may be other undiscovered mechanisms by which trastuzumab induces regression in cancer.

Additionally, somatic mutations of receptors from the ErbB family in the tumour, which are commonly clustered in the tyrosine kinase domain of the receptor (exons 18 to 21) or high polysomy of the ErbB, gene may be of positive predictive influence^{2,3}. However, there are patients described, in whose tumours no EGFR-mutation could be found despite showing responses to erlotinib⁴. Therefore, the inventors of the present invention have assumed that besides changes in ErbB other genetic phenomenon might exist in tumours sensitive to ErbB inhibitors.

Activation or overexpression of HER-2/neu is often associated with up-regulation of the vascular endothelial growth factor (VEGF) in cancerous tissue.

VEGF is an important signaling protein involved in both vasculogenesis and angiogenesis. VEGF activity has been mostly studied on cells of the vascular endothelium, although it does have effects on a number of other cell types (e.g. stimulation monocyte/macrophage migration, neurons, cancer cells, kidney epithelial cells ). *In vitro,* VEGF has been shown to stimulate endothelial cell mitogenesis and cell migration. VEGF is also a vasodilator and increases microvascular permeability and was originally referred to as vascular permeability factor.

All members of the VEGF family stimulate cellular responses by binding to tyrosine kinase receptors disposed on the cell surface, causing them to dimerize and become activated through transphosphorylation, although to different sites, times and extents. These VEGF receptors have an extracellular portion consisting of 7 immunoglobulin-like domains, a single transmembrane spanning region and an intracellular portion containing a split tyrosine-kinase domain. VEGF-A binds to VEGFR-1 (Flt-1) and VEGFR-2 (KDR/Flk-1). VEGFR-2 appears to mediate almost all of the known cellular responses to VEGF. The function of VEGFR-1 is less well defined, although it is thought to modulate VEGFR-2 signaling. Another function of VEGFR-1 may be to act as a dummy/decoy receptor, sequestering VEGF from VEGFR-2 binding (this appears to be particularly important during vasculogenesis in the embryo). VEGF-C and VEGF-D, but not VEGF-A, are ligands for a third receptor (VEGFR-3), which mediates lymphangiogenesis.

VEGF ligands have been implicated with poor prognosis in breast cancer. Numerous studies show a decreased survival rate in tumors overexpressing VEGF ligands. The overexpression of VEGF may be an early step in the process of metastasis, a step that is involved in the "angiogenic" switch. Although VEGF ligands have been correlated with poor survival, its exact mechanism of action in the progression of tumors remains unclear.

Studies indicate that an association between HER-2/neu and VEGF predicts clinical outcome in primary breast cancer patients⁶. It was found that the positive association between HER-2/*neu* and VEGF expression implicates VEGF in the aggressive phenotype exhibited by HER-2/*neu* overexpression, and supports the use of combination therapies directed against both HER-2/neu and VEGF for treatment of breast cancers that overexpress HER-2/*neu*.
As ErbB-2/Her-2/neu is a member of the ErbB receptor family, it can be assumed that the above mentioned mechanisms are also applicable to the other ErbB receptors introduced above.

VEGFA is a member of the VEGF family (Vascular endothelial growth factor) family, and related to the PDGF (Platelet derived growth factor) and FGF (Fibrobast growth factor) family, it can be assumed that the above mentioned mechanisms are also applicable to the other growth factors of said families.

The above study has been carried out by measuring HER-2/neu and VEGF using the ELISA method in primary breast tumor tissue lysates. This means that, in the said study, the expression levels of HER-2/neu and VEGF haven been determined on the protein level, with use of suitable antibodies.

The above study has been carried out with primary breast tumor tissue lysates from a cohort of about 600 unselected patients. For this purpose, Breast cancer tissue specimens were selected by a pathologist at the time of surgery and stored at -70°C until use. Frozen tissue samples of 100-200 mg were then pulverized with a microdismembrator prior to the ELISA protocol.

In clinical practice, however, tissue samples taken from a cancer patient are treated with formaline and paraffine right after biopsy, in order to conserve them for later immunohistochemical examination. This standard treatment, however, renders the said tissue samples unsuitable for later examination with the ELISA method.

Moreover, the authors of the above study have reported that some isoforms of VEGF were not detectable in about 35 % of the samples, which they explain with poor sensitivity of the ELISA assay, which is obviously not sufficient to detect very low levels of VEGF expression.

Another approach, namely FISH (Fluorescence In Situ Hybridization), has severe drawbacks as well.

Besides labor intensity it mainly requires a defined protocol of tissue fixation and conservation that is not applicable to routine diagnostics, where tissue are differently handled according to time point of tissue drawing. In Best case scenarios⁹, the agreement rate between different labs is at about 92%. For example time to fixation and time to fixation tremendously impact the diagnostic result. The fixation buffer and importantly the temperature during Paraffin embedding affect the In-Situ Hybridization results, particularly for RNA measurements.

Other approaches, like immunohistochemical staining procedures (IHC), which are considered as gold standard in cancer diagnostics, have only poor sensitivity⁹ and 79% inter-lab reproducibility in best case scenarios (e.g. Her-2/neu Test by FDA approved DAKO system).

For VEGFA determination, the variabilities are much higher and and there is currently no reliable VEGFA test being validated having clinical impact for decision making in breast cancer diagnostics.

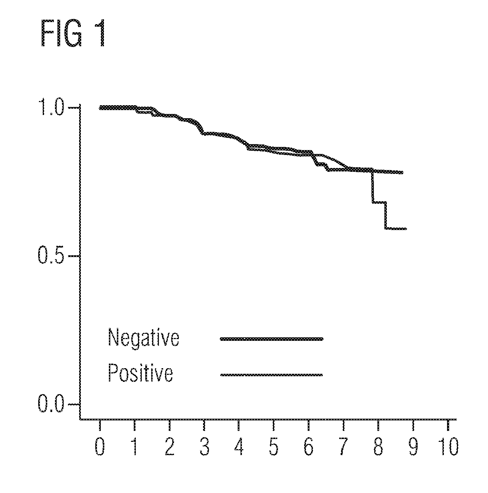

In summary, current testing does not allow a differentiation between Her-2/neu positive and negative tumors, and/or VEGFA positive and negative tumors (see Fig. 1). This means that, for the patients affected, a promising therapy option (i,e, anti VEGF and/or Anti ErbB therapy) is lost as patients which would benefit from such therapy can not be determined.

One additional reason for the poor performance of IHC in this case can be seen in the fact that protein expression and mRNA amount are only moderately correlating (r=0,5 - 0,8; p<0,0001) and yet not identical.

Moreover VEGFA is difficult to determine on the protein level because of frequent alternative splicing events and different protein half lifes that cannot be resolved by immuno assay experiments. There are at least 6 Isoforms of VEGFA, each being expressed to varying extent in different tissues.

Moreover, the large isoforms (e.g. VEGFA with 206 kDA) may have a different diffusion pattern than small isoforms (e.g. VEGFA with 165 kDA). As VEGFA is a soluble and secreted factor diffusion of small isoforms from its place of synthesis corrupts the analysis of VEGFA expression on protein basis. Similarly the protein expression of Her-2/neu is affected by proteolytic cleavage of the extracellular membrane and variable times of receptor internalization and degradation. This also explains conflicting data when measuring Her-2/neu protein by antibodies detecting the intra- or extracellular portion.

Moreover the protein stability of certain VEGFA splice forms alleviate the impact of other splice variants indicating response to anti-angiogenic drugs.

Besides, the multiplexing capabilities of ELISA method as well as of IHC and FISH are quite restricted in view of limits tissue material available. It is thus not possible to determine a larger number of analytes in one and the same sample.

We thus conclude that standard methods based on the determination of the protein level of at least one gene encoding for a ligand from the Vascular endothelial growth factor (VEGF) family and of at least one gene encoding for a receptor from the ErbB-family in said sample
a) are not sensitive enough to resolve Her-2/neu positive and negative tumors, and/or VEGFA positive and negative tumors
b) can only be used with fresh tissue or frozen tissue samples (i.e. not with samples obtained by standard methods),
c) have only none or limited multiplexing capabilizties, and/or
d) do not allow the differentiation between diferent VEGF Isoforms.

### Definitions

It is to be noted that, herein, the terms "expression level of a protein, e.g. a ligand and/or a receptor" and "expression level of a gene encoding for a protein, e.g. a ligand and/or a receptor" are used synonymously.

The term "determining the expression level of a gene/protein on a non protein basis" relates to methods which are not focussed on the secondary gene translation products, i.e proteins, but on other levels of the gene expression, based on RNA and DNA analysis. In one embodiment of this invention the analysis uses mRNA including its precursor froms. In yet another embodiment the detection of methylation patterns and transcription factor footprints in gene regulatory regions such as promoter structures are used.

The term "prediction", as used herein, relates to an individual assessment of the malignancy of a tumor, or to the expected survival rate (DFS, disease free survival) of a patient, if the tumor is treated with a given therapy. In contrast thereto, the term "prognosis" relates to an individual assesment of the malignancy of a tumor, or to the expected survival rate (DFS, disease free survival) of a patient, if the tumor remains untreated.

The term "response marker" relates to a marker which can be used to predict the clinical response of a patient towards a given treatment. Response includes direct observation of tumor shrinkage upon neoadjuvant or palliative treatment as evident by e.g. CT-Scans and/or serum biomarkers as well as effects on Disease Free Survival (DFS), Overall Survival (OAS), Metastasis Specific Survival (MSS), Disease Specific Survival and related assessments.

The term "neoplastic lesion" or "neoplastic disease" or "neoplasia" refers to a cancerous tissue this includes carcinomas, (e.g., carcinoma in situ, invasive carcinoma, metastatic carcinoma) and pre-malignant conditions, neomorphic changes independent of their histological origin (e.g. ductal, lobular, medullary, mixed origin). The term "cancer" as used herein includes carcinomas, (e.g., carcinoma in situ, invasive carcinoma, metastatic carcinoma) and pre-malignant conditions, neomorphic changes independent of their histological origin. The term "cancer" is not limited to any stage, grade, histomorphological feature, invasiveness, agressivity or malignancy of an affected tissue or cell aggregation. In particular stage 0 cancer, stage I cancer, stage II cancer, stage III cancer, stage IV cancer, grade I cancer, grade II cancer, grade III cancer, malignant cancer, primary carcinomas, and all other types of cancers, malignancies and transformations associated with female organs, particularly breast cancer, are included. Particularly types of adenocarcinoma are included, as well as all carcinomas of unknown primary (cup-syndroms). The terms "neoplastic lesion" or "neoplastic disease" or "neoplasia" or "cancer" are not limited to any tissue or cell type they also include primary, secondary or metastatic lesions of cancer patients, and also comprises lymph nodes affected by cancer cells or minimal residual disease cells either locally deposited (e.g. bone marrow, liver, kidney) or freely floating throughout the patients body.

The term "neoplastic cells" refer to abnormal cells that grow by cellular proliferation more rapidly than normal. As such, neoplastic cells of the invention may be cells of a benign neoplasm or may be cells of a malignant neoplasm.

Furthermore, the term "characterizing the state of a neoplastic disease" is related to, but not limited to, measurements and assessment of one or more of the following conditions: Type of tumor, histomorphological appearance, dependence on external signal (e.g. hormones, growth factors), invasiveness, motility, state by TNM (2) or similar, agressivity, malignancy, metastatic potential, and responsiveness to a given therapy.

The terms "biological sample" or "clinical sample", as used herein, refer to a sample obtained from a patient. The sample may be of any biological tissue or fluid. Such samples include, but are not limited to, sputum, blood, serum, plasma, blood cells (e.g., white cells), tissue, core or fine needle biopsy samples, cell-containing body fluids, free floating nucleic acids, urine, peritoneal fluid, and pleural fluid, liquor cerebrospinalis, tear fluid, or cells there from. Biological samples may also include sections of tissues such as frozen or fixed sections taken for histological purposes or microdissected cells or extracellular parts thereof. A biological sample to be analyzed is tissue material from a neoplastic lesion taken by aspiration or punctuation, excision or by any other surgical method leading to biopsy or resected cellular material. Such a biological sample may comprise cells obtained from a patient. The cells may be found in a cell "smear" collected, for example, by a nipple aspiration, ductal lavarge, fine needle biopsy or from provoked or spontaneous nipple discharge. In another embodiment, the sample is a body fluid. Such fluids include, for example, blood fluids, serum, plasma, lymph, ascitic fluids, gynecological fluids, or urine but not limited to these fluids.

The term "therapy modality", "therapy mode", "regimen" or "chemo regimen" as well as "therapy regimen" refers to a timely sequential or simultaneous administration of antitumor, aand/or anti vascular, and/or immune stimulating, and/or blood cell proliferative agents, and/or radiation therapy, and/or hyperthermia, and/or hypothermia for cancer therapy. The administration of these can be performed in an adjuvant and/or neoadjuvant mode. The composition of such "protocol" may vary in the dose of the single agent, time-frame of application and frequency of administration within a defined therapy window. Currently various combinations of various drugs and/or physical methods, and various schedules are under investigation.

By "array" or "matrix" is meant an arrangement of addressable locations or "addresses" on a device. The locations can be arranged in two dimensional arrays, three dimensional arrays, or other matrix formats. The number of locations can range from several to at least hundreds of thousands. Most importantly, each location represents a totally independent reaction site. Arrays include but are not limited to nucleic acid arrays, protein arrays and antibody arrays. A "nucleic acid array" refers to an array containing nucleic acid probes, such as oligonucleotides, polynucleotides or larger portions of genes. The nucleic acid on the array is preferably single stranded. Arrays wherein the probes are oligonucleotides are referred to as "oligonucleotide arrays" or "oligonucleotide chips." A "microarray," herein also refers to a "biochip" or "biological chip", an array of regions having a density of discrete regions of at least about 100/cm², and preferably at least about 1000/cm². The regions in a microarray have typical dimensions, e.g., diameters, in the range of between about 10-250 µm, and are separated from other regions in the array by about the same distance. A "protein array" refers to an array containing polypeptide probes or protein probes which can be in native form or denatured. An "antibody array" refers to an array containing antibodies which include but are not limited to monoclonal antibodies (e.g. from a mouse), chimeric antibodies, humanized antibodies or phage antibodies and single chain antibodies as well as fragments from antibodies.
The term "small molecule", as used herein, is meant to refer to a compound which has a molecular weight of less than about 5 kD and most preferably less than about 4 kD. Small molecules can be nucleic acids, peptides, polypeptides, peptidomimetics, carbohydrates, lipids or other organic (carbon-containing) or inorganic molecules. Many pharmaceutical companies have extensive libraries of chemical and/or biological mixtures, often fungal, bacterial, or algal extracts, which can be screened with any of the assays of the invention to identify compounds that modulate a bioactivity.

The terms "modulated" or "modulation" or "regulated" or "regulation" and "differentially regulated" as used herein refer to both upregulation [i.e., activation or stimulation (e.g., by agonizing or potentiating] and down regulation [i.e., inhibition or suppression (e.g., by antagonizing, decreasing or inhibiting)].

The term "transcriptome" relates to the set of all messenger RNA (mRNA) molecules, or "transcripts", produced in one or a population of cells. This also includes performs of the messenger RNA as well as non-translated RNA molecules or fragments thereof. The term can be applied to the total set of transcripts in a given organism, or to the specific subset of transcripts present in a particular cell type. Unlike the genome, which is roughly fixed for a given cell line (excluding mutations), the transcriptome can vary with external environmental conditions. Because it includes all *mRNA* transcripts in the cell, the transcriptome reflects the genes that are being actively expressed at any given time, with the exception of mRNA degradation phenomena such as transcriptional attenuation. The discipline of transcriptomics examines the expression level of mRNAs in a given cell population, often using high-throughput techniques based on DNA microarray technology.

The term "expression levels" refers, e.g., to a determined level of gene expression. The term "pattern of expression levels" refers to a determined level of gene expression compared either to a reference gene (e.g. housekeeper or inversely regulated genes) or to a computed average expression value (e.g. in DNA-chip analyses). A pattern is not limited to the comparison of two genes but is more related to multiple comparisons of genes to reference genes or samples. A certain "pattern of expression levels" may also result and be determined by comparison and measurement of several genes disclosed hereafter and display the relative abundance of these transcripts to each other.

Alternatively, a differentially expressed gene disclosed herein may be used in methods for identifying reagents and compounds and uses of these reagents and compounds for the treatment of cancer as well as methods of treatment. The differential regulation of the gene is not limited to a specific cancer cell type or clone, but rather displays the interplay of cancer cells, muscle cells, stromal cells, connective tissue cells, other epithelial cells, endothelial cells of blood vessels as well as cells of the immune system (e.g. lymphocytes, macrophages, killer cells).

A "reference pattern of expression levels", within the meaning of the invention shall be understood as being any pattern of expression levels that can be used for the comparison to another pattern of expression levels. In a preferred embodiment of the invention, a reference pattern of expression levels is, e.g., an average pattern of expression levels observed in a group of healthy or diseased individuals, serving as a reference group.

"Primer pairs" and "probes", within the meaning of the invention, shall have the ordinary meaning of this term which is well known to the person skilled in the art of molecular biology. In a preferred embodiment of the invention "primer pairs" and "probes", shall be understood as being polynucleotide molecules having a sequence identical, complementary, homologous, or homologous to the complement of regions of a target polynucleotide which is to be detected or quantified. In yet another embodiment nucleotide analogues are also comprised for usage as primers and/or probes.

"Individually labeled probes", within the meaning of the invention, shall be understood as being molecular probes comprising a polynucleotide, oligonucleotide or nucleotide analogue and a label, helpful in the detection or quantification of the probe. Preferred labels are fluorescent molecules, luminescent molecules, radioactive molecules, enzymatic molecules and/or quenching molecules.

"Arrayed probes", within the meaning of the invention, shall be understood as being a collection of immobilized probes, preferably in an orderly arrangement. In a preferred embodiment of the invention, the individual "arrayed probes" can be identified by their respective position on the solid support, e.g., on a "chip".

The phrase "tumor response", "therapeutic success", or "response to therapy" refers, in the adjuvant chemotherapeutic setting to the observation of a defined tumor free or recurrence free survival time (e.g. 2 years, 4 years, 5 years, 10 years). This time period of disease free survival may vary among the different tumor entities but is sufficiently longer than the average time period in which most of the recurrences appear. In a neo-adjuvant therapy modality, response may be monitored by measurement of tumor shrinkage and regression due to apoptosis and necrosis of the tumor mass.

The term "recurrence" or " recurrent disease" includes distant metastasis that can appear even many years after the initial diagnosis and therapy of a tumor, or local events such as infiltration of tumor cells into regional lymph nodes, or occurrence of tumor cells at the same site and organ of origin within an appropriate time.

"Prediction of recurrence" or "prediction of therapeutic success" does refer to the methods described in this invention. Wherein a tumor specimen is analyzed for it's gene expression and furthermore classified based on correlation of the expression pattern to known ones from reference samples. This classification may either result in the statement that such given tumor will develop recurrence and therefore is considered as a "non responding" tumor to the given therapy, or may result in a classification as a tumor with a prolonged disease free post therapy time.

"Biological activity" or "bioactivity" or "activity" or "biological function", which are used interchangeably, herein mean an effector or antigenic function that is directly or indirectly exerted by a polypeptide (whether in its native or denatured conformation), or by any fragment thereof *in vivo* or *in vitro.* Biological activities include but are not limited to binding to polypeptides, binding to other proteins or molecules, enzymatic activity, signal transduction, activity as a DNA binding protein, as a transcription regulator, ability to bind damaged DNA, etc. A bioactivity can be modulated by directly affecting the subject polypeptide. Alternatively, a bioactivity can be altered by modulating the level of the polypeptide, such as by modulating expression of the corresponding gene.

The term "marker" or "biomarker" refers to a biological molecule, e.g., a nucleic acid, peptide, protein, hormone, etc., whose presence or concentration can be detected and correlated with a known condition, such as a disease state.

The term "ligand", as used herein, relates to a molecule that is able to bind to and form a complex with a biomolecule to serve a biological purpose. In a narrower sense, it is an effector molecule binding to a site on a target protein, by intermolecular forces such as ionic bonds, hydrogen bonds and Van der Waals forces. The docking (association) is usually reversible (dissociation). Actual irreversible covalent binding between a ligand and its target molecule is rare in biological systems. Ligand binding to receptors often alters the chemical conformation, i.e. the three dimensional shape of the receptor protein. The conformational state of a receptor protein determines the functional state of a receptor. The tendency or strength of binding is called affinity. Ligands include substrates, inhibitors, activators, and neurotransmitters.

The term "agonist", as used herein, relates to a substance that binds to a specific receptor and triggers a response in the cell. It mimics the action of an endogenous ligand that binds to the same receptor.

The term "receptor", as used herein, relates to a protein on the cell membrane or within the cytoplasm or cell nucleus that binds to a specific molecule (a ligand), such as a neurotransmitter, hormone, or other substance, and initiates the cellular response to the ligand. Ligand-induced changes in the behavior of receptor proteins result in physiological changes that constitute the biological actions of the ligands.

The term "signalling pathway" is related to any intra- or intercellular process by which cells converts one kind of signal or stimulus into another, most often involving ordered sequences of biochemical reactions out- and inside the cell, that are carried out by enzymes and linked through homones and growth factors (intercellular), as well as second messengers (intracellular), the latter resulting in what is thought of as a "second messenger pathway". In many signalling pathways, the number of proteins and other molecules participating in these events increases as the process eminates from the initial stimulus, resulting in a "signal cascade" and often results in a relatively small stimulus eliciting a large response.

The term "marker gene," as used herein, refers to a differentially expressed gene whose expression pattern may be utilized as part of a predictive, prognostic or diagnostic process in malignant neoplasia or cancer evaluation, or which, alternatively, may be used in methods for identifying compounds useful for the treatment or prevention of malignant neoplasia and breast cancer in particular. A marker gene may also have the characteristics of a target gene.

"Target gene", as used herein, refers to a differentially expressed gene involved in cancer, e.g. breast cancer in a manner in which modulation of the level of the target gene expression or of the target gene product activity may act to ameliorate symptoms of malignant neoplasia and lung, ovarian, cervix, esophagus, stomach, pancreas, prostate, head and neck, renal cell, colorectal or breast cancer in particular. A target gene may also have the characteristics of a marker gene.

The term "expression level", as used herein, relates to the process by which a gene's DNA sequence is converted into functional protein (i.e. ligands) and particularly to the amount of said conversion. However, expression level also refers to non-translated RNA molecules, which may effect other genes and/or gene products.

The term "hybridization based method", as used herein, refers to methods imparting a process of combining complementary, single-stranded nucleic acids or nucleotide analogues into a single double stranded molecule. Nucleotides or nucleotide analogues will bind to their complement under normal conditions, so two perfectly complementary strands will bind to each other readily. In bioanalytics, very often labeled, single stranded probes are in order to find complementary target sequences. If such sequences exist in the sample, the probes will hybridize to said sequences which can then be detected due to the label. Other hybridization based methods comprise microarray and/or biochip methods. Therein, probes are immobilized on a solid phase, which is then exposed to a sample. If complementary nucleic acids exist in the sample, these will hybridize to the probes and can thus be detected. These approaches are also known as "array based methods". Yet another hybridization based method is PCR, which is described below. When it comes to the determination of expression levels, hybridization based methods may for example be used to determine the amount of mRNA for a given gene.

The term "a PCR based method" as used herein refers to methods comprising a polymerase chain reaction (PCR). This is an approach for exponentially amplifying nucleic acids, like DNA or RNA, via enzymatic replication, without using a living organism. As PCR is an in vitro technique, it can be performed without restrictions on the form of DNA, and it can be extensively modified to perform a wide array of genetic manipulations. When it comes to the determination of expression levels, a PCR based method may for example be used to detect the presence of a given mRNA by (1) reverse transcription of the complete mRNA pool (the so called transcriptome) into cDNA with help of a reverse transcriptase enzyme, and (2) detecting the presence of a given cDNA with help of respective primers. This approach is commonly known as reverse transcriptase PCR (rtPCR)

The term "determining the protein level", as used herein, refers to methods which allow the quantitative and/or qualitative determination of one or more proteins in a sample. These methods include, among others, protein purification, including ultracentrifugation, precipitation and chromatography, as well as protein analysis and determination, including the use protein microarrays, two-hybrid screening, blotting methods including western blot, one- and two dimensional gelelectrophoresis, isoelectric focusing and the like.

The term "method based on the electrochemical detection of molecules" relates to methods which make use of an electrode system to which molecules, particularly biomolecules like proteins, nucleic acids, antigens, antibodies and the like, bind under creation of a detectable signal. Such methods are for example disclosed in W00242759, W00241992 and W002097413 filed by the applicant of the present invention, the content of which is incorporated by reference herein. These detectors comprise a substrate with a planar surface which is formed, for example, by the crystallographic surface of a silicon chip, and electrical detectors which may adopt, for example, the shape of interdigital electrodes or a two dimensional electrode array. These electrodes carry probe molecules, e.g. nucleic acid probes, capable of binding specifically to target molecules, e.g. target nucleic acid molecules. The probe molecules are for example immobilized by a Thiol-Gold-binding. For this purpose, the probe is modified at its 5'-or 3'-end with a thiol group which binds to the electrode comprising a gold surface. These target nucleic acid molecules may carry, for example, an enzyme label, like horseradish peroxidise (HRP) or alkaline phosphatase. After the target molecules have bound to the probes, a substrate is then added (e.g., α-naphthyl phosphate or 3,3'5,5'-tetramethylbenzidine which is converted by said enzyme, particularly in a redox-reaction. The product of said reaction, or a current generated in said reaction due to an exchange of electrons, can then be detected with help of the electrical detector in a site specific manner.

The term "anamnesis" relates to patient data gained by a physician or other healthcare professional by asking specific questions, either of the patient or of other people who know the person and can give suitable information (in this case, it is sometimes called heteroanamnesis), with the aim of obtaining information useful in formulating a diagnosis and providing medical care to the patient. This kind of information is called the symptoms, in contrast with clinical signs, which are ascertained by direct examination.

The term "etiopathology" relates to the course of a disease, that is its duration, its clinical symptoms, and its outcome.

The term "detection of a ligand and/or receptor" as used herein means both the qualitative detection of the presence of the respective gene as well as the quantitative detect detection of of the expression level of the respective gene, e.g. by quantitative reverse transcriptase PCR.

The term "nucleic acid molecule" is intended to indicate any single- or double stranded nucleic acid molecule comprising DNA (cDNA and/or genomic DNA), RNA (preferably mRNA), PNA, LNA and/or Morpholino.

The term "stringent conditions" relates to conditions under which a probe will hybridize to its target subsequence, but to no other sequences. Stringent conditions are sequence-dependent and will be different in different circumstances. Longer sequences hybridize specifically at higher temperatures. Generally, stringent conditions are selected to be about 5° C. lower than the thermal melting point (Tm) for the specific sequence at a defined ionic strength and pH. The Tm is the temperature (under defined ionic strength, pH and nucleic acid concentration) at which 50% of the probes complementary to the target sequence hybridize to the target sequence at equilibrium. (As the target sequences are generally present in excess, at Tm, 50% of the probes are occupied at equilibrium). Typically, stringent conditions will be those in which the salt concentration is less than about 1.0 M Na ion, typically about 0.01 to 1.0 M Na ion (or other salts) at pH 7.0 to 8.3 and the temperature is at least about 30° C. for short probes (e.g. 10 to 50 nucleotides) and at least about 60° C. for longer probes. Stringent conditions may also be achieved with the addition of destabilizing agents, such as formamide and the like.

The term "fragment of the nucleic acid molecule" is intended to indicate a nucleic acid comprising a subset of a nucleic acid molecule according to one of the claimed sequences. The same is applicable to the term "fraction of the nucleic acid molecule".

The term "variant of the nucleic acid molecule" refers herein to a nucleic acid molecule which is substantially similar in structure and biological activity to a nucleic acid molecule according to one of the claimed sequences.

The term "homologue of the nucleic acid molecule" refers to a nucleic acid molecule the sequence of which has one or more nucleotides added, deleted, substituted or otherwise chemically modified in comparison to a nucleic acid molecule according to one of the claimed sequences, provided always that the homologue retains substantially the same binding properties as the latter.

The term "derivative," as used herein, refers to a nucleic acid molecule that has similar binding characteristics to a target nucleic acid sequence as a nucleic acid molecule according to one of the claimed sequences

### Object of the invention

It is one object of the present invention to provide a method for the determination of tumors which are characterized by elevated expression and/or overexpression of a receptor belonging to the ErbB family and/or a ligand belonging to the VEGF-family.

It is another object of the present invention to provide an in vivo method for the determination of tumor tissue which is characterized by elevated expression and/or overexpression of a receptor belonging to the ErbB family and/or a ligand belonging to the VEGF-family.

It is yet another object of the present invention to provide an in vitro and/or in vivo method for the determination whether or not a tumor is likely to be susceptible to a medication related to the signalling pathway of a receptor belonging to the ErbB family and/or a ligand belonging to the VEGF-family.

It is another object of the present invention to overcome the above determined disadvantages of respective methods based on an ELISA approach, FISH and/or IHC.

In particular, it is an obeject of the present invention to provide a method which
a) is sensitive enough to resolve Her-2/neu positive and negative tumors, and/or VEGFA positive and negative tumors
b) can be used samples obtained by standard methods, e.g. formalin fixed paraffin embedded samples),
c) has multiplexing capabilities, and/or
d) allows the differentiation between diferent VEGF Isoforms.

These objects are met with methods and means according to the independent claims of the present invention. The dependent claims are related to preferred embodiments.

### Summary of the invention

Before the invention is described in detail, it is to be understood that this invention is not limited to the particular component parts of the devices described or process steps of the methods described as such devices and methods may vary. It is also to be understood that the terminology used herein is for purposes of describing particular embodiments only, and is not intended to be limiting. It must be noted that, as used in the specification and the appended claims, the singular forms "a," "an" and "the" include singular and/or plural referents unless the context clearly dictates otherwise. It is moreover to be understood that, in case parameter ranges are given which are delimited by numeric values, the ranges are deemed to include these limitation values.

According to the invention, a method is provided for predicting a clinical response of a patient suffering from or at risk of developing a neoplastic disease towards a given mode of treatment, said method comprising the steps of:
a) obtaining a biological sample from said patient;
b) determining, on a non protein basis, the expression level of at least one gene encoding for a ligand from the Vascular endothelial growth factor (VEGF) family and of and of at least one gene encoding for a receptor from the ErbB-family, or a gene co-expressed therewith, in said patient,
c) comparing the pattern of expression levels determined in (b) with one or several reference pattern(s) of expression levels; and
d) predicting therapeutic success for said given mode of treatment in said patient or implementing therapeutic regimen targeting the signalling pathway of said ligand and/or receptor is related to in said patient from the outcome of the comparison in step (c).

It is to be understood that genes which are co-expressed with a given target gene, like a gene encoding for a receptor from the receptor from the ErbB-family, may be used in addition tp, or even as a substitute for, said target gene.

In a preferred embodiment of the present invention, the detection of the at least one ligand from the Vascular endothelial growth factor (VEGF) and the at least one gene encoding for a receptor from the ErbB-family, or a gene co-expressed therewith, is done on a non protein basis.

In this regard it is to be understood, that the detection of these markers on protein basis has, surprisingly, turned out to be inferior to the detection of these markers on a RNA basis for several reasons.

One reason is the fact that the therapeutic downregulation of target protein activity and/or target protein amount as exemplified by usage of antibody regimen such as Herceptin and/or Avastin has different effect on tumor cells having identical protein content but different RNA levels.

For example, the downregulation of VEGFA protein can be better compensated by cells having higher levels of RNA transcript resulting in faster reproduction and synthesis of target protein. This tumorbiological difference cannot be assessed by protein analysis such as immunohistochemistry.

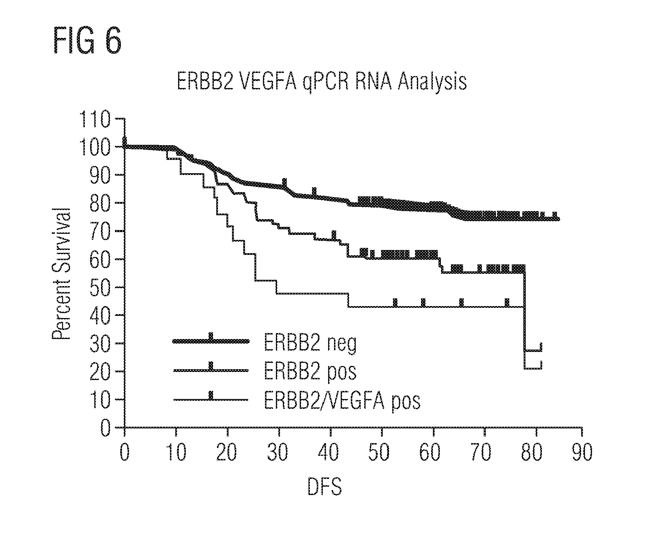

Another reason, besides technical insufficiencies as described above, is the fact that posttranslational events and protein modifications, particularly enzymatic cleavage by proteinases, affect the detection by antibodies and mask the clinically relevant marker expression visible by RNA determination and result in false negative adjustments (see Figure 6).

In a preferred embodiment of the present invention, it is provided that the mode of treatment for which prediction is sought is a treatment related to the signalling pathway of a ligand from the Vascular endothelial growth factor (VEGF) family and/or a treatment related to the signalling pathway of a receptor from the ErbB-family.

The ErbB family of receptors comprises four closely related receptor tyrosine kinases, namely
- EGFR (ErbB-1) (epidermal growth factor receptor), also known as ErbB-1 or HER1
- HER2 (ErbB-2; Her-2/neu),
- HER3 (ErbB-3), and
- HER4 (ErbB-4).
   There is evidence that any of these receptors may be related to cancer genesis, as well as to an enhanced expression of VEGF ligands and/or VEGFR receptors.

The VEGF family of growth factor ligands comprises several members which all have in common that they feature a cystine-knot domain, and bind to tyrosine kinase receptors, like those from the ErbB family. The VEGF family comprises, apart from the vascular endothelial growth factors (VEGF), the Placenta growth factor (PlGF), as well as Platelet derived growth factors (PDGF). Particularly, the following growth factors belong to the VEGF family:
- VEGF-A,
- VEGF-B,
- VEGF-C,
- VEGF-D (FIGF),
- PDGF-A,
- PDGF-B,
- PDGF-C, and/or
- PlGF.

A number of other VEGF-related proteins have also been discovered encoded by viruses (VEGF-E) and in the venom of some snakes (VEGF-F).

All of these growth factors are ligands which are related to the ErbB signalling pathway, as their expression level is upregulated upon activation or self activation of a receptor of the ErbB family, particularly of EGFR. The growths factors do thus meet the above identified definition according to which the said ligand is related to the signalling pathway of receptors from the ErbB receptor family.

The above mentioned targets, i.e. receptor genes and/or ligand genes the expression level of which is used for predicting therapeutic success for said given mode of treatment according to the present invention are listed in Table 1.

In another preferred embodiment of the present invention, it is provided that at least one of the said ligand genes the expression level of which is determined is VEGF-A and/or at least one of the receptor genes the expression level of which is determined is Her-2/neu.

As an alternative to Her-2/neu, genes co-expressed therewith may be determined as well. Such genes are for example located on the 17q12 chromosome region and the 8q24 chromosome region.

The respective genes are for example enumerated in EP1365034, the content of which shall be incorporated herein by reference. This reference includes in particular the genes co-expressed with Her-2/neu.

In a preferred embodiment genes co-expressed with Her-2/neu, are selected from the group consisting of MGC9753, GRB7, THRA, RARA, and TOP02A.

Basically, an altered expression level of either of the aformenetioned agents can have different reasons, these being
- gene amplification of an oncogene (frequently seen in Her-2/neu)
- overexpression of the respective gene due to altered Methylation pattern
- altered properties of a transcription factor, a promotor or another factor which leads to an upregulation of the expression level of the said agent.

Her-2/neu (also known as ErbB-2, ERBB2) is a member of the ErbB protein family, more commonly known as the epidermal growth factor receptor family. HER-2/neu is notable for its role in the pathogenesis of breast cancer and as a target of treatment. It is a cell membrane surface-bound receptor tyrosine kinase and is normally involved in the signal transduction pathways leading to cell growth and differentiation. HER2 is thought to be an orphan receptor, with none of the EGF family of ligands able to activate it. However, ErbB receptors dimerise on ligand binding, and HER2 is the preferential dimerisation partner of other members of the ErbB family. The HER2 gene is a proto-oncogene located at the long arm of human chromosome 17(17q11.2-q12).

Approximately 25-30 percent of breast cancers have an amplification of the HER-2/neu gene or overexpression of its protein product. Overexpression and/or gene amplification of this receptor in breast cancer is associated with increased disease recurrence and worse prognosis. Vascular endothelial growth factor (VEGF) is an important signaling protein involved in both vasculogenesis (the de novo formation of the embryonic circulatory system) and angiogenesis (the growth of blood vessels from pre-existing vasculature). As its name implies, VEGF activity has been mostly studied on cells of the vascular endothelium, although it does have effects on a number of other cell types (e.g. stimulation monocyte/macrophage migration, neurons, cancer cells, kidney epithelial cells ). In vitro, VEGF has been shown to stimulate endothelial cell mitogenesis and cell migration. VEGF is also a vasodilator and increases microvascular permeability and was originally referred to as vascular permeability factor.

VEGF-A has been implicated with poor prognosis in breast cancer. Numerous studies show a decreased OS and DFS in those tumors overexpressing VEGF-A. The overexpression of VEGF-A may be an early step in the process of metastasis, a step that is involved in the "angiogenic" switch. Although VEGF-A has been correlated with poor survival, its exact mechanism of action in the progression of tumors remains unclear.

In yet another preferred embodiment of the present invention, it is yet provided that the method according to the invention comprises the additional step of:

### e) determining the expression level of a gene encoding for a Growth factor Receptor-Bound Protein (GRB).

In an even more preferred embodiment, the expression level of a gene encoding for Growth factor Receptor-Bound Protein 7, which is an SH2-domain adaptor protein that binds to Receptor-tyrosine kinases and provides the intra-cellular direct link to the Ras proto-oncogene, is determined. Human GRB7 is located on the long arm of chromosome 17, next to the ERBB2 (alias Her-2/neu) proto-oncogene.

In another preferred embodiment of the present invention, it is provided that upregulated expression of at least one ligand and/or receptor determined in step (b) is indicative of a promising prediction as regards therapeutic success for a mode of treatment or therapeutic regimen related to the signalling pathway of a ligand from the Vascular endothelial growth factor (VEGF) family and/or of a receptor from the ErbB-family.

In this context, other parameters may as well be used and combined in order to predict the therapeutic success for said given mode of treatment. The parameters may be chosen from the group consisting of
- Menopausal status
- Overall histological state
- ECOG performance status
- Serum Her-2/neu level
- Serum VEGFA level
- Serum EGFR level
- LDH serum levels

The ECOG performance status is used by doctors and researchers to assess how a patient's disease is progressing, assess how the disease affects the daily living abilities of the patient, and determine appropriate treatment and prognosis⁵.

In yet another preferred embodiment of the present invention, it is provided that said given mode of treatment (a) acts on recruitment of lymphatic vessels, angiogenesis, cell proliferation, cell survival and/or cell motility, and/or b) comprises administration of a chemotherapeutic agent.

Furthermore, it is provided in an another preferred embodiment of the present invention that said given mode of treatment comprises, in addition, chemotherapy, administration of small molecule inhibitors, antibody based regimen, anti-proliferation regimen, pro-apoptotic regimen, pro-differentiation regimen, radiation and/or surgical therapy.

Said chemotherapy may comprise the administration of at least one agent selected from the group consisting of Cyclophosphamid (Endoxan®, Cyclostin®). Adriamycin (Doxorubicin) (Adriblastin®), BCNU (Carmustin) (Carmubris®), Busulfan (Myleran®),Bleomycin (Bleomycin®), Carboplatin (Carboplat®), Chlorambucil (Leukeran®), Cis-Platin (Cisplatin®), Platinex (Platiblastin®), Dacarbazin (DTIC®;Detimedac®), Docetaxel (Taxotere®), Epirubicin (Farmorubicin®), Etoposid (Vepesid®), 5-Fluorouracil (Fluroblastin®, Fluorouracil®), Gemcitabin (Gemzar®), Ifosfamid (Holoxan®), Interferon alpha (Roferon®), Irinotecan (CPT 11, Campto®), Melphalan (Alkeran®),Methotrexat (Methotrexat®, Farmitrexat®), Mitomycin C (Mitomycin®), Mitoxantron (Novantron®), Oxaliplatin (Eloxatine®), Paclitaxel (Taxol®), Prednimustin (Sterecyt®), Procarbazin (Natulan®), Pemetrexed (Alimta®), Ralitrexed (Tomudex®), Topotecan (Hycantin®), Trofosfamid (Ixoten®), Vinblastin (Velbe®), Vincristin (Vincristin®), Vindesin (Eldisine®) and/or Vinorelbin (Navelbine®). The person skilled in the art will, from scientific textbooks, databases and literature, be able to choose other chemotherapeutic agents which are suitable in this context, without requiring an inventive step.

In yet another preferred embodiment of the present invention, it is provided that said given mode of treatment or therapeutic regimen related to the signalling pathway of said ligand from the VEGF family and/or receptor from the Erb-B family comprises adminsitration of at least one agent selected from the group consisting of:
- an agonist of said ligand
- an agonist of a ligand specific for said receptor
- an antagonist, e.g. an antibody or an antibody fragment, against said ligand and/or receptor,
- an antisense nucleic acid inhibiting the expression of a gene encoding for a said ligand and/or receptor,
- a small molecular drug,
- a kinase inhibitor specific for the given receptor,
- spefifically binding proteins, and/or
- phages.

Such spefifically binding proteins are for example Cullines, Lectins or Ankyrins, or fragments, repeating units or derivatives thereof.

By way of illustration and not by way of restriction said agents may be selected from the group consisting of

| Target | Agonist/antagonist | Kinase inhibtors |
|---|---|---|
| Her-2/neu (ErbB-2) | Herceptin (Trastuzumab) | Lapatinib (Tykerb) GW572016 |
| | Pertuzumab | AEE-788 |
| | | CI-1033 |
| VEGF-A | Avastin (Bevacizumab) | Sunitinib (Sutent)* |
| | 2C3 | Sorafenib (Nexavar) * |
| | VEGF-trap (AVE-0005) | Axitinib* |
| | Ranibizumab (Lucentis) | Pazopanib* |

| | | |
|---|---|---|
| *these agents are inhibtors of receptors binding VEGF-A Other potential agents may be selected from the group consisting of Cetuximab (tradename Erbitux®, target receptor is EGFR), Matuzumab (EMD7200, target receptor is EGFR), Trastuzumab (tradename Herceptin®, target receptor is HER2/neu), Pertuzumab (target receptor is HER2/neu), Bevacizumab (tradename Avastin®, target ligand is VEGFA), 2C3 (target ligand is VEGFA), VEGF-trap (AVE-0005, target ligands are VEGFA and PIGF), IMC-1121B (target receptor is VEGFR2), CDP-791 (target receptor is VEGFR2), Gefitinib (tradename Iressa®, ZD-1839, target receptor is EGFR), Erlotinib (tradename Tarceva®, OSI-774, target receptor is EGFR), EKB-569 (target receptor is EGFR), PKI-166 (target receptor is EGFR), ), PKI-166 (target receptor is EGFR), Lapatinib (tradename tycerb®, target receptor is EGFR and Her-2/neu), GW572016 (target receptors are EGFR and Her-2/neu), AEE-788 (target receptors are EGFR, Her-2/neu and VEGFR-2), CI-1033 (target receptors are EGFR, Her-2/neu and Her4), AZD6474 (target receptors are EGFR and VEGFR-2). | | |

However, other treatments related to the ErbB receptor family signalling pathway which fall under the scope of the present invention comprise the administration of Sorafenib (tradename Nexavar®, BAY 43-9005, target receptors are VEGFR-2, VEGFR-3, c-KIT, PDGFR-B, RET and Raf-Kinase), BAY 57-9352 (target receptor is VEGFR-2), Sunitinib (tradename Sutent®, target receptors are VEGFR-1, VEGFR-2 and PDGFR), AG13925 (target receptors are VEGFR-1 and VEGFR-2), AG013736 (target receptors are VEGFR-1 and VEGFR-2), AZD2171 (target receptors are VEGFR-1 and VEGFR-2), ZD6474 (target receptors are VEGFR-1, VEGFR-2 and VEGFR-3), Vandetenib (ZD 7646), Vatalanib PTK-787/ZK-222584 (target receptors are VEGFR-1 and VEGFR-2), CEP-7055 (target receptors are VEGFR-1, VEGFR-2 and VEGFR-3), CP-547 (target receptors are VEGFR-1 and VEGFR-2), CP-632 (target receptors are VEGFR-1 and VEGFR-2), GW786024 (target receptors are VEGFR-1, VEGFR-2 and VEGFR-3), AMG706 (target receptors are VEGFR-1, VEGFR-2 and VEGFR-3), Imatinib mesylate (tradename Glivec®/Gleevec®, target receptors are bcr-abl and c-KIT), BMS-214662 (target enzyme is Ras farnesyl transferase), CCI-779 (target enzyme is mTOR), RAD0001 (tradename everolismus®, target enzyme is mTOR), CI-1040 (target enzyme is MEK), SU6668 (target receptors are VEGFR-2, PDGFR-B and FGFR-1), AZD6126, CP547632 (target receptors are VEGFRs), CP868596 GW786034 (target receptors are PDGFRs), ABT-869 (target receptors are VEGFRs and PDGFRs), AEE788 (target receptors are VEGFRs and PDGFRs), AZD0530 (target enzymes are src and abl), and CEP7055.

In a preferred embodiment the said treatment comprises the administration of the therapeutics Herceptin, Lapatinib, VEGF trap and Avastin.

In a particularly preferred embodiment the said treatment comprises the administration of the therapeutics Herceptin and Avastin.

Such a combined treatment is beneficial of tumors which are characterized by an elevated expression level and/or gene copy number of Her-2/neu, elevated expression level and/or gene copy number of co-amplified genes located on chromosome 17q12 such as MGC9753 and/or THRA and/or TOP02A, reduced expression level of EGFR expression, high expression level of VEGFC and high expression level of VEGF-A isoforms.

However, such a combined treatment is particularly beneficial for the treatment of tumors which are characterized by an elevated expression level of Her-2/neu and VEGF-A.

Clinical studies have shown that a combined therapy targeting the HER-2/neu proto-oncogene and the vascular endothelial growth factor with Herceptin (trastuzumab) and Avastin (bevacizumab) as first line treatment in breast cancer patients in which HER2-amplification has been diagnosed by fluorescence in situ hybridization (FISH), leads to increased survial rates in the patients involved in the study⁷.

In another embodiment of the present invention, a method of selecting a therapy modality for a patient afflicted with a neoplastic disease is provided, said method comprising the steps of:
a) obtaining a biological sample from said patient;
b) predicting from said sample, by the method according to the above, therapeutic success for a plurality of individual modes of treatment; and
c) selecting a mode of treatment which is predicted to be successful in step (b).
   This means that, for example, the invention provides the possibility to specifically determine with high sensitivity whether or not a neoplastic disease in a patient characterized by an elevated expression level of Erb-B and/or VEGF, particularly preferred of an elevated expression level of Her-2/neu and VEGF-A.

On the basis of this finding a therapy can be selected which is most promising for the respective patient, e.g. an anti-Erb-B and/or anti-VEGF-A treatment, like the administration of the therapeutics Herceptin and Avastin.

In addition the inventors suggest, for the first time, to use this finding for the decision whether or not Herceptin and/or Lapatinib should be used as a combination partner for the anti-VEGF therapies depicted above. In this regard, the accurate detection of Her-2/neu and EGFR enables to identify a subpopulation of tumors that co-overexpresses both receptors, yet having a comparatively low expression of Her-2/neu, and yet detectable coamplification of neighbouring genes of chromosome 17q12 that cannot be resolved by immunohistochemical techniques. This subpopulation is particularly sensitive to Lapatinib.

Moreover, the inventors suggest, for the first time, to use this finding for the decision whether can be used to decide wether Avastin and/or Erbitux should be used as a combination partner for the anti-Her-2/neu therapies depicted above. In this regard, the accurate detection of Her-/neu and EGFR enables to identify a subpopulation of tumors that co-overexpresses both receptors, yet having a comparatively low expression of Her-2/neu that cannot be resolved by immunohistochemical techniques.

The inventors suggest moreover, for the first time, to use this finding for the decision In addition this finding can be used to decide wether Herceptin and/or Erbitux should be used as a combination partner for the the anti-VEGF therapies depicted above.

Moreover this finding also enables to decide which patients should receive cytotoxic chemotherapeutic regimen such as anthracyclin and/or taxane and/or regimen listed above, as the chemotherapeutic regimen functions in part via its anti-angiogenic activity, which results from the proliferation blockade of endothelial cells attracted and/or activated by VEGF factors.

In other words, the method according to the invention helps to detect those tumors which are most susceptible to a combined anti-Erb-B and/or anti-VEGF treatment. These tumors have so far remained undetected with methods from the state of the art.

In a preferred embodiment, said method comprises the steps of
a) obtaining a sample comprising cancer cells from said patient;
b) separately maintaining aliquots of the sample in the presence of one or more test compositions;
c) comparing expression of a single or plurality of molecules, selected from the ligands and/or receptors listed in Table 1 in each of the aliquots; and
d) selecting a test composition which induces a lower level of expression of ligands and/or receptors from Table 1 and/or a higher level of expression of ligands and/or receptors from Table 1 in the aliquot containing that test composition, relative to the level of expression of each ligand in the aliquots containing the other test compositions.

It is particularly preferred that, in the method according to the invention, the said expression level is determined by
a) a hybridization based method;
b) a PCR based method;
c) a method based on the electrochemical detection of particular molecules, and/or by
d) an array based method.

The above mentioned methods have in common that they are focussed on the detection of nucleic acids, particularly on the detection of mRNA, DNA, PNA, LNA and/or Morpholino. Moreover, these methods provide the option to determine more than two agents at the same time ("multiplexing"). Therefore, not only the expression levels of one ligand from the Vascular endothelial growth factor (VEGF) family and/or of one receptor from the ErbB-family can be determined, but the expression level of many other genes of interest, like the above mentioned Growth factor Receptor-Bound Protein (GRB), other ligands, receptors, oncogenes or metabolism related genes can be determined in order to better characterize a given cancer or neoplastic disease in a patient.

Applicant's unpublished data suggests that the above shown phenomena (i.e. survival expectancy dependent on whether or not the tumor is ErbB-positive/negative and/or VEGFA positive ornegative, and the conclusions for anti ErbB-herapy and/or anti VEGF therapy) are not only applicable for breast caner, but for other gynoaecological cancer types as well.

Therefore, in a preferred embodiment of the present invention it is provided that said cancer or neoplastic disease is selected from the group consisting of gynaecological cancers including Breast cancer, Ovarian cancer, Cervical cancer, Endometrial cancer, Vulval cancer, and the like.

In yet another preferred embodiment of the present invention it is provided that that the expression level of at least one of the said ligands and/or receptors is determined with RT-PCR (reverse transcriptase polymerase chain reaction) of the ligand and/or receptor related mRNA.

In yet another preferred embodiment of the present invention it is provided that the gene copy number of at least one of the said ligands and/or receptors is determined with PCR (polymerase chain reaction) of the ligand and/or receptor related DNA sequence and/or genomic regions located nearby said genes and a reference gene that is preferably located in an unaltered region of the genome, most preferably on the same chromosome arm.

In another preferred embodiment of the present invention, it is provided that the expression level of at least one of the said ligands of is determined in fixed and/or paraffin embedded tissue samples.

For this purpose, at least one fixative may used in a preferred embodiment which is selected from the group consisting of Neutral Buffered Formaline, Unbuffered Formaline, Ethanol, Acetone, Methanol, Methacarn, Carnoy's fixative, AFA-Fixative (Formaldehyde, Ethanol and acetic acid), Pen-Fix (alcoholic formalin fixative), Glyo-Fixx (glyoxal-based fixative), Hope (Hepes-glutamic acid buffer mediated organic solvent fixative), and/or Zinc Formal-Fixx (Formaldehyde fixative which contains zinc).

In yet another preferred embodiment of the present invention, it is provided that the expression level of at least one of the said ligands or receptors is determined in serum, plasma or whole blood samples.

Routinely, in tumor diagnosis tissue samples are taken as biopsies form a patient and undergo diagnostic procedures. For this purpose, the samples are fixed in formaline and/or parrafine and are then examined with immunohistochemistry methods. The formaline treatment leads to the inactivation of enzymes, as for example the ubiquitous RNA-digesting enzymes (RNAses). For this reason, the mRNA status of the tissue (the so called transcriptome), remains unaffected.

However, the formaline treatment leads to partial depolymerization of the individual mRNA molecules. Same applies for other fixatives, as for example mentioned in the above enumeration. For this reason, the current doctrine is that fixed tissue samples can not be used for the analysis of the transcriptome of said tissue.

For this reason, it is provided in a preferred embodiment of the present invention that after lysis, the samples are treated with silica-coated magnetic particles and a chaotropic salt, in order to purify the nucleic acids contained in said sample for further determination.

Collaborators of the inventors of the present invention have developed an approach which however allows successful purification of mRNA out of tissue samples fixed in such manner, and which is disclosed, among others, in W003058649, WO2006136314A1 and DE10201084A1, the content of which is incorporated herein by reference.

Said method comprises the use of magnetic particles coated with silica (SiO₂). The silica layer is closed and tight and is characterized by having an extremely small thickness on the scale of a few nanometers. These particles are produced by an improved method that leads to a product having a closed silica layer and thus entail a highly improved purity. The said method prevents an uncontrolled formation of aggregates and clusters of silicates on the magnetite surface whereby positively influencing the additional cited properties and biological applications. The said magnetic particles exhibit an optimized magnetization and suspension behavior as well as a very advantageous run-off behavior from plastic surfaces. These highly pure magnetic particles coated with silicon dioxide are used for isolating nucleic acids, including DNA and RNA, from cell and tissue samples, the separating out from a sample matrix ensuing by means of magnetic fields. These particles are particularly well-suited for the automatic purification of nucleic acids, mostly from biological body samples for the purpose of detecting them with different amplification methods.

The selective binding of these nucleic acids to the surface of said particles is due to the affinity of negatively charged nucleic acids to silica containing media in the presence of chaotropic salts like guanidinisothiocyanate. Said binding properties are known as the so called "boom principle". They are described in the European patent EP819696, the content of which is incorporated herein by reference.

The said approach is particularly useful for the purification of mRNA out of formaline and/or paraffine fixed tissue samples. In contrast to most other approaches, which leave very small fragments behind that are not suitable for later determination by PCR and/or hybridization technologies, the said approach creates mRNA fragments which are large enough to allow specific primer hybridzation and/or specific probe hybridization. A minimal size of at least 100 bp, more preferably 200 base pairs is needed for specific and robust detection of target gene expression. Moreover it is also necessary to not have too many inter-sample variations with regard to the size of the RNA fragments to guarantee comparability of gene expression results. Other issues of perturbance of expression data by sample preparation problems relate to the contamination level with DNA, which is lower compared to other bead based technologies. This of particular importance, as the inventors have observed, that DNAse treatment is not efficient in approximately 10% of FFPE samples generated by standard procedures and stored at room temperature for some years before cutting and RNA extraction.

The said approach thus allows a highly specific determination of candidate gene expression levels with one of the above introduced methods, particularly with hybridization based methods, PCR based methods and/or array based methods, even in formaline and/or paraffine fixed tissue samples, and is thus extremely beneficial in the context of the present invention, as it allows the use of tissue samples fixid with formaline and/or paraffine, which are available in tissue banks and connected to clinical databases of sufficient follow-up to allow retrospective analysis.

Another important aspect is that the said approach allows the simultaneaous determination of more than one analyte (multiplexing), and is thus ideally suited for the determination of, among others, at least one ligand from the Vascular endothelial growth factor (VEGF) family and of and of at least one gene encoding for a receptor from the ErbB-family, or a gene co-expressed therewith, in said sample, as provided by the method according to the present invention.

Because of these capabilities, the expression level of other analytes may be determined as well, in order to enhance the prediction accuracy. Such analytes are for example Growth factor Receptor-Bound Protein (GRB), which is discussed above, or coamplified genes on 17q12 und 8q24.

Another advantage is that thereby the expression level of a housekeeping gene can be determined simultaneously. By this approach one can derive a calibration factor in order to normalize the expression values of the target genes in samples which have different shares of tumor tissue and non tumor tissue. Preferably this also enables the detection of lymphoid cells infiltrating the tumor site and effecting particularly the antibody based regimen.

In contrast thereto, the multiplexing capabilities of IHC, ELISA and FISH methods are quite limited due to cross reactions in the different binding procedures, and additional need for chemical additives.

Furthermore, a kit useful for carrying out one of the said methods is provided, said kit comprising at least
a) a primer pair and/or a probe each having a sequence sufficiently complementary to a gene encoding for a ligand from the VEGF family and/or a receptor from the ErbB family and/or
b) at least an antibody directed against a ligand from the VEGF family and/or a receptor from the ErbB-family.

In yet another embodiment of the invention a method for correlating the clinical outcome of a patient suffering from or at risk of developing a neoplastic disease is provided, said disease being charcterized by the presence or non-presence of a defect in expression of a ligand from the VEGF family and/or a receptor from the ErbB-family, said method comprising the steps of:
a) obtaining a fixed biological sample from said patient;
b) determining the expression level of at least one gene encoding for a ligand from the VEGF family and/or one receptor from the ErbB-family in said patient according to any of the above methods, and
c) correlating the pattern of expression levels determined in (b) with said patient's data, said data being selected from the group consisting of etiopathology data, clinical symptoms, anamnesis data and/or data concerning the therapeutic regimen.
   The said method is particularly beneficial for epidemiological studies. These studies profit from the fact that large tissue databases exist comprising paraffin and/or formalin fixed tissue samples together with an extensive documentation of the patient's history, including etiopathology data, clinical symptoms, anamnesis data and/or data concerning the therapeutic regimen.

The said methods allows for large scale studies which comprise the correlation of the clinical outcome of a patient suffering from or at risk of developing a neoplastic disease with the presence or non-presence of a defect in ErbB receptor expression and/or VEGF ligand expression. In order to successfully adopt this approach, the above introduced method for mRNA purification comprising silica coated magnetic beads and chaotropic salts is quite helpful.

Furthermore, the present invention provides a nucleic acid molecule, selected from the group consisting of
a) the nucleic acid molecule presented as SEQ ID NO:1 - 66
b) a nucleic acid molecule having a length of 4 - 80 nucleotides, preferably 18 - 30 nucleotides, the sequence of which corresponds to the sequence of a single stranded fragment of a gene encoding for a ligand and/or receptor selected from the group consisting of VEGFA, VEGFB, VEGFC, FIGF/VEGFD, EGFR/HER-1, ERBB2/Her-2/neu/HER-2, ERBB3/HER-3, ERBB4/HER-4, MGC9753, GRB7, THRA, RARA, and/or TOP02A
c) a nucleic acid molecule that is a fraction, variant, homologue, derivative, or fragment of the nucleic acid molecule presented as SEQ ID NO: 1 - 66
d) a nucleic acid molecule that is capable of hybridizing to any of the nucleic acid molecules of a) - c) under stringent conditions
e) a nucleic acid molecule that is capable of hybridizing to the complement of any of the nucleic acid molecules of a) - d) under stringent conditions
f) a nucleic acid molecule that is capable of hybridizing to the complement of a nucleic acid molecule of e)
g) a nucleic acid molecule having a sequence identity of at least 95 % with any of the nucleic acid molecules of a) - f)
h) a nucleic acid molecule having a sequence identity of at least 70 % with any of the nucleic acid molecules of a) - f)
i) a complement of any of the nucleic acid molecules of a) - h), or
   i) a nucleic acid molecule that comprises any nucleic acid molecule of a) - i).

VEGFA, VEGFB, VEGFC, FIGF / VEGFD, EGFR / HER-1, ERBB2 / Her-2/neu / HER-2, ERBB3 / HER-3, ERBB4 / HER-4 are genes related to ligands from the Vascular endothelial growth factor (VEGF) family or to receptors from the ErbB-family.

MGC9753, GRB7, THRA, RARA, and TOP02A are genes which are co-expressed with Her-2/neu. Their determination may thus replace the determination of Her-2/neu.

See Table 1 for a sequence listing. These nucleic acids are being used either as primers for a polymerase chain reaction protocol, or as detectable probes for monitoring the said process.

Furthermore it is provided that the said nucleic acid is selected from the group consisting of DNA, RNA, PNA, LNA and/or Morpholino. The nucleic acid may, in a preferred embodiment, be labelled with at least one detectable marker. This feature is applicable particularly for those nucleic acids which serve as detectable probes for monitoring the polymerase chain reaction process

Such detectable markers may for example comprise at least one label selected from the group consisting of fluorescent molecules, luminescent molecules, radioactive molecules, enzymatic molecules and/or quenching molecules.

In a particularly preferred embodiment, the said detectable probes are labeled with a fluorescent marker at one end and a quencher of fluorescence at the opposite end of the probe. The close proximity of the reporter to the quencher prevents detection of its fluorescence; breakdown of the probe by the 5' to 3' exonuclease activity of the taq polymerase breaks the reporter-quencher proximity and thus allows unquenched emission of fluorescence, which can be detected. An increase in the product targeted by the reporter probe at each PCR cycle therefore causes a proportional increase in fluorescence due to the breakdown of the probe and release of the reporter.

In another preferred embodiment of the present invention, a kit of primers and/or detection probes is provided, comprising at least one of the nucleic acids according to the above enumeration and/or their fractions, variants, homologues, derivatives, fragments, complements, hybridizing counterparts, or molecules sharing a sequence identity of at least 70%, preferably 95 %.

Said kit may, in another preferred embodiment, comprise at least one of the nucleic acid molecules presented as SEQ ID NO: 1 - 66, and/or their fractions, variants, homologues, derivatives, fragments, complements, hybridizing counterparts, or molecules sharing a sequence identity of at least 70%, preferably 95 %, for the detection of at least one gene encoding for a ligand from the VEGF family and/or at least one gene encoding for a receptor from the ErbB-family.

Furthermore, the use of a nucleic acid according as recited above, or of a kit as recited above for the prediction of a clinical response of a patient suffering from or at risk of developing a neoplastic disease towards a given mode of treatment.

### Disclaimer

To provide a comprehensive disclosure without unduly lengthening the specification, the applicant hereby incorporates by reference each of the patents and patent applications referenced above.

The particular combinations of elements and features in the above detailed embodiments are exemplary only; the interchanging and substitution of these teachings with other teachings in this and the patents/applications incorporated by reference are also expressly contemplated. As those skilled in the art will recognize, variations, modifications, and other implementations of what is described herein can occur to those of ordinary skill in the art without departing from the spirit and the scope of the invention as claimed. Accordingly, the foregoing description is by way of example only and is not intended as limiting. The invention's scope is defined in the following claims and the equivalents thereto. Furthermore, reference signs used in the description and claims do not limit the scope of the invention as claimed.

### Brief description of the examples and drawings

Additional details, features, characteristics and advantages of the object of the invention are disclosed in the subclaims, and the following description of the respective figures and examples, which, in an exemplary fashion, show preferred embodiments of the present invention. However, these drawings should by no means be understood as to limit the scope of the invention.

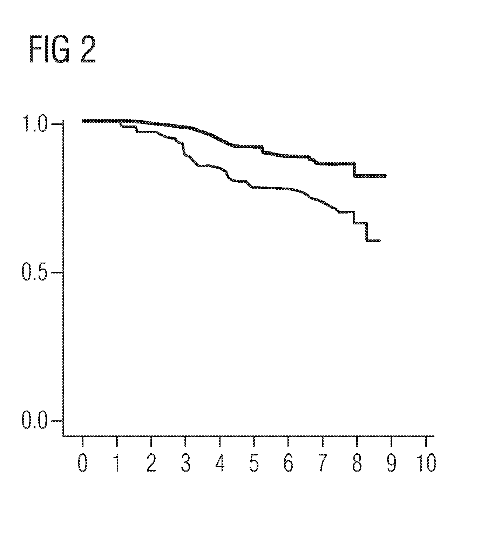

Fig. 1 and 2 shows, in two Kaplan meyer curves, the effect of VEGF-A overexpression on the overall survival of patients suffering from high risk primary Breast tumors, as assessed by IHC (Immunohistochemistry, Fig. 1) and a PCR approach according to the present invention (Fig. 2). In the graphs, the probablity that a patient survives is plotted versus time (here: months).

For the IHC approach (Fig. 1), VEGF-A levels were assessed on a protein basis in 227 of the patients, using a monoclonal antibody (Neomarkers, Fremont, CA). Immunoreactivity was evaluated in the neoplastic epithelial cells using a combined score System based on the suni of the staining intensity (0=negative staining, 1=weak, 2=intermediate, 3=strong staining) and the percentage of positive cells (0=0%, 1 = 1-25%, 2=26-50%, 3=>50%). Scores from 0 to 3 were given for the staining intensity and the percentage of positive cells, these then being added together to obtain the overall score with a maximum of 6.

In contrast thereto, for the approach involving the method acording to the invention (Fig. 2) RNA was isolated from 281 formalin-fixed paraffin-embedded ("FFPE") tumor tissue samples employing an experimental method based on proprietary magnetic beads from Siemens Medical Solutions Diagnostics, followed by kinetic one-step RT-PCR for assessment of mRNA expression. A total of 40 cycles of RNA amplification were applied and the cycle threshold (CT) of the target genes was identified. CT scores were normalized by subtracting the CT score of the housekeeping gene from the CT score of the target gene (Delta CT). RNA results were then reported as 40-Delta CT scores, which would correlate proportionally to the mRNA expression level of the target gene.

Basically, high levels of VEGF-A mRNA were found to be a significant negative prognostic factor for overall survival (OS) (HR=2.30, p=0.004; adjusted for treatment: HR=2.31, p=0.004).

A comparison between Fig. 1 and Fig. 2 shows that standard IHC methods do not suffice to assess the VEGF-A status in the above patient cohort. This means that patients suffering from a VEGF A positive tumor (i.e. a tumor characzterized by VEGF A overexpression) will have no access to possible therapeutic approaches, i.e anti VEGF therapy with Avastin or the like, although such therapy might turn out highly beneficial.

In contrast thereto, VEGF-A mRNA over-expression, as assessed by a method according to the present invention, is a more accurate negative prognostic factor for OS and DFS in high-risk breast cancer patients, compared to increased VEGF-A protein levels assessed by IHC. This means that this method provides the option to differentiate between VEGF-positive and negative tumors. Therefore, patients suffering from a tumor characterized by VEGF-A overexpression (i.e VEGF-A positive), can be detected, and prepared for anti-VEGF-a therapy.

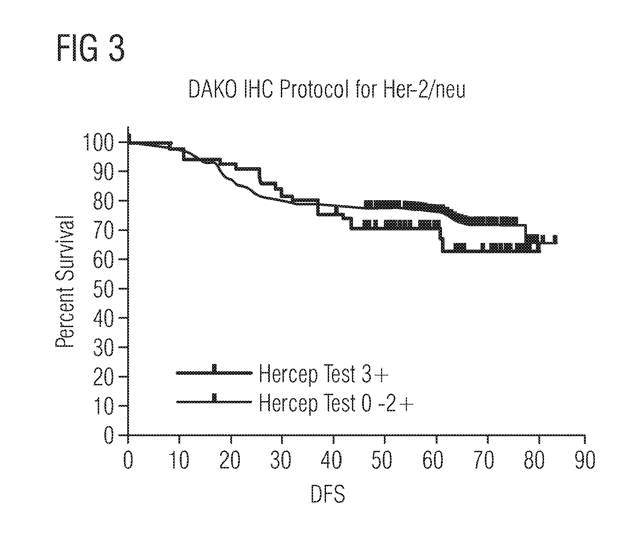

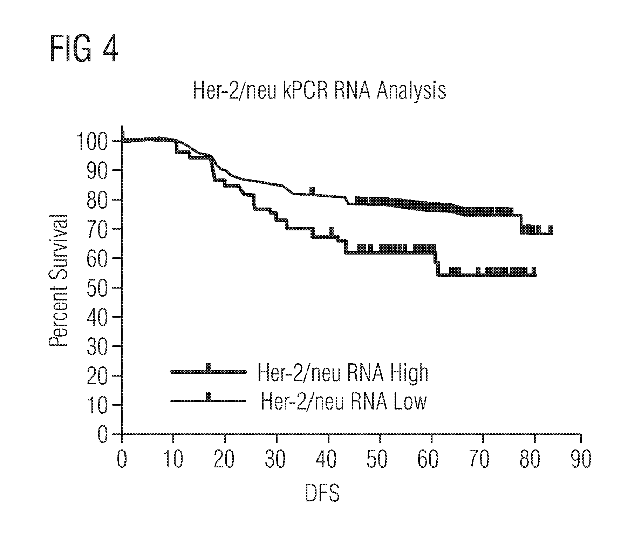

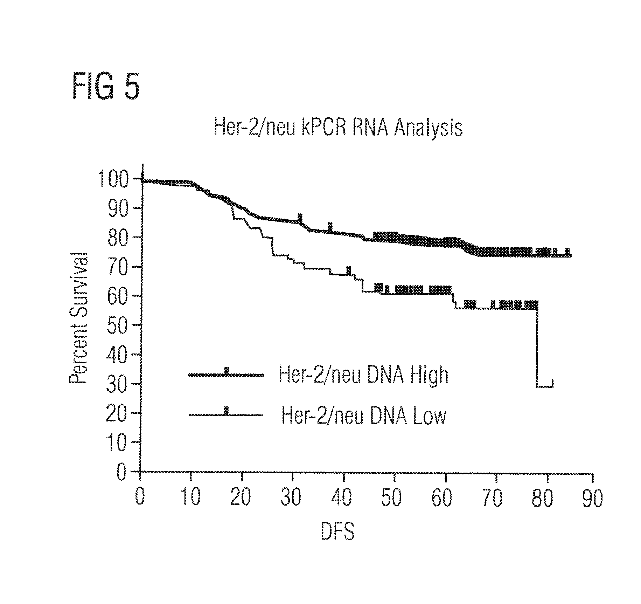

Fig. 3, 4 and 5 show, in three Kaplan meyer curves, the effect of VEGF-A overexpression on the overall survival of patients suffering from high risk primary breast tumors, as assessed by IHC (Immunohistochemistry, Fig. 3) and a PCR approach according to the present invention (Fig. 4 and 5). In the graphs, the probablity that a patient survives is plotted versus time (here: months).

For the IHC approach (Fig. 1), VEGF-A levels were assessed on a protein basis in 286 of the patients, using the FDA approved, commercially available DAKO kit on a Ventana autostainer, which can be regarded as best practice of the conventional IHC approach. Immunoreactivity was evaluated in the neoplastic epithelial cells using the DAKO Score System based on the the staining intensity and localization (DAKO 0=negative staining, DAKO 1=weak, DAKO 2=intermediate and interspersed membrane staining, 3=strong and continous membrane staining) according to manufacturers instructions and by two independent pathologists.

In contrast thereto, for the approach involving the method acording to the invention (Fig. 4) RNA was isolated from 281 formalin-fixed paraffin-embedded ("FFPE") tumor tissue samples employing an experimental method based on proprietary magnetic beads from Siemens Medical Solutions Diagnostics, followed by kinetic one-step RT-PCR for assessment of mRNA expression. A total of 40 cycles of RNA amplification were applied and the cycle threshold (CT) of the target genes was identified. CT scores were normalized by subtracting the CT score of the housekeeping gene from the CT score of the target gene (Delta CT). RNA results were then reported as 2^{40-Delta CT} scores, which would correlate proportionally to the mRNA expression level of the target gene.

Basically, high levels of Her-2/neu mRNA were found to be a significant negative prognostic factor for disease free survival (DFS) (HR=7.13, p=0.0076).

In contrast thereto, for the approach involving the method acording to the invention (Fig. 4) RNA was isolated from 281 formalin-fixed paraffin-embedded ("FFPE") tumor tissue samples employing an experimental method based on proprietary magnetic beads from Siemens Medical Solutions Diagnostics, followed by kinetic one-step RT-PCR for assessment of mRNA expression. A total of 40 cycles of DNA amplification were applied and the cycle threshold (CT) of the target genes was identified. CT scores were normalized by subtracting the CT score of the reference gene (i.e. MMP28 located nearby the chromosomal region of Her-2/neu, but yet not be effected by the genomic alteration) from the CT score of the target gene (Delta CT). RNA results were then reported as 2^{40-Delta CT} scores, which would correlate proportionally to the mRNA expression level of the target gene.

Basically, gene copy number of Her-2/neu DNA were found to be a significant negative prognostic factor for disease free survival (DFS) (HR=8.357, p=0.0038).

A comparison between Fig. 3 and Fig. 4 as well as Fig. 5 show that standard IHC methods do not suffice to assess the Her-2/neu status in the above patient cohort. This means that patients suffering from a Her-2/neu positive tumor (i.e. a tumor characterized by Her-2/neu overexpression and/or elevated gene copy numbers) will have no access to possible therapeutic approaches, i.e anti Her-2/neu therapy with Herceptin, Lapatinib or the like, although such therapy might turn out highly beneficial.

Fig. 6 shows, in a Kaplan Meyer curve, the effect of ErbB2 (= Her-2/neu) overexpression and VEGF-A overexpression on the death free survival rate of patients suffering from high risk primary Breast tumors, as assessed by PCR approach according to the present invention (Fig. 2). In the graph, the percentage of surviving patients is plotted versus time (here: months).

The shown data are unpublished data of the inventors of the present invention. Patients selected for this study had undergone breast surgery and were under adjuvant anthracycline-based dose-dense sequential chemotherapy, i.e Epirubicin followed by CMF (combined treatment with epirubicin, cyclophosphamide, methotrexate and fluorouracil) with or without Paclitaxel.

Results of the Cox multivariate regression analysis for DFS revealed that, in the presence of treatment group (p=0.90), HER-2 over-expression was related to a significantly increased risk for disease progression [HR (hazard ratio)=1.65, 95% Cl (confidence interval): 1.05-2.60, p=0.03], while VEGF-A over-expression was not (HR=1.50, 95% Cl: 0.97-2.32, p=0.07). In contrast, when looking at overall survival (OS) after adjusting for treatment (p=0.98), only VEGF-A over-expression was related to significantly poorer prognosis (HR=2.24, 95% Cl: 1.27-3.94, p=0.005), while HER-2 over-expression was not (HR=1.70, 95% Cl: 0.98-2.97, p=0.06), probably due to the treatment of many of these patients with Herceptin after disease progression. Over-expression of both HER-2 and VEGF-A was observed in 36 of the 266 patients (13.5%) and was found to be a significant negative prognostic factor for both DFS (HR=2.46, 95% Cl: 1.32-4.58, p=0.005) and OS (HR=3.81, 95% Cl: 1.76-8.24, p=0.001).

It is obvious that patients the tumor of which has been determined to be Her-2/neu negative have a much better expectation to survive than those the tumor of which has been determined to be Her-2/neu negative, and VEGFA positive, respectively.

This again means that those patients with poor expectation to survive would draw substantial benefit from anti-ErbB treatment e.g. Herceptin®, Lapatinib®, Tarceva®) and/or anti VEGFA treatment, anti VEGFR treatment (e.g. Sutent®, Sorafenib®) and anti-VEGF treatment (e.g. Avastin®) regimen.

It should be clear from the above that the shown differentiation is not possible with IHC methods, only with the method according to the present invention.

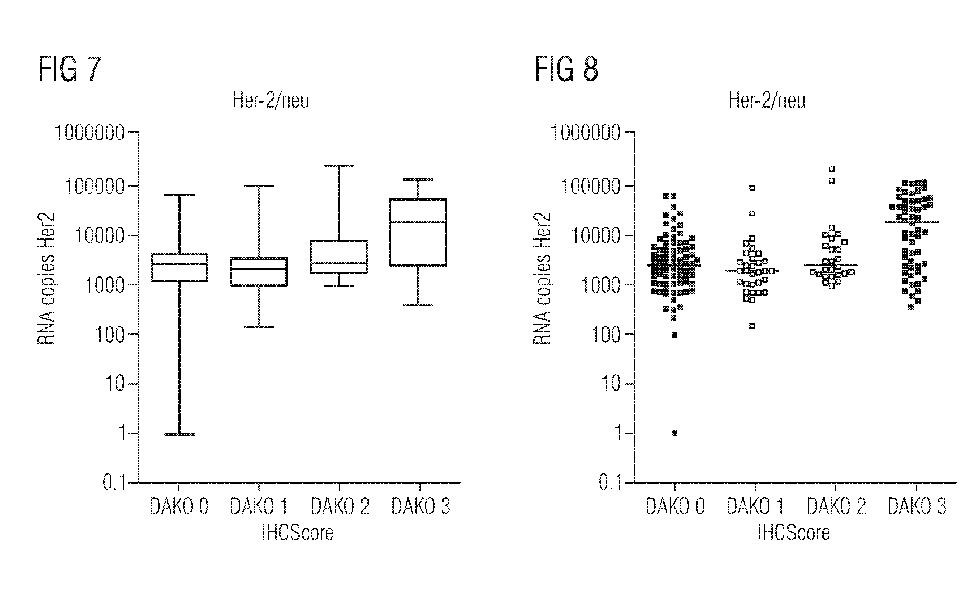

Figs. 7 and 8 depict, in two different plotting schemes, the RNA expression level of Her-2/neu (y-Axis) as described above compared to the current gold standard technology, the DAKO Hercep Test^{™} on a Ventana staining automate (x-Axis). As can be seen the lack of significance of the IHC methodology depicted in Fig. 3 is due to both false negative Her-2/neu determinations (tumors being characterized by DAKO 0, DAKO 1 or DAKO 2 but exhibiting a high Her-2/neu expression) and false positive determinations (tumors being characterized by DAKO 3 but exhibiting a low Her-2/neu expression). While the false positive tumors are currently overtreated by addition of Herceptin® to standard chemotherapeutic treatment yet exposing the patients to cardiotoxic side effects, the false negative patients do not receive this potentially life saving regimen.

**Table 1: Genes of Interest**

| Gene_Symbol [A] | Ref. Sequences Description [A] | Ref. Sequence [A] | Locus_Link_ID [A] | Unigene_ID [A] | OMIN [A] |
|---|---|---|---|---|---|
| VEGFA | Vascular endothelial growth factor | NM_003376 | 7422 | 73793 | 192240 |
| VEGFB | Vascular endothelial growth factor B | NM_003377 | 7423 | 78781 | 601398 |
| VEGFC | Vascular endothelial growth factor C prepro-protein | NM_005429 | 7424 | 79141 | 601528 |
| FIGF / VEGFD | Vascular endothelial growth factor D prepro-protein | NM_004469 | | 11392 | 300091 |
| EGFR / HER-1 | epidermal growth factor receptor (erythroblastic leukemia viral (v-erb-b) oncogene homolog, avian) | NM_005228 | 1956 | 77432 | 131550 |
| ERBB2 / Her-2/neu / HER-2 | v-erb-b2 erythroblastic leukemia viral oncogene homolog 2, neuro/glioblasto ma derived oncogene homolog | NM_004448 | 2064 | 323910 | 164870 |
| ERBB3 / HER-3 | v-erb-b2 erythroblastic leukemia viral oncogene homolog 3 | NM_001982 | 2065 | 199067 | 190151 |
| ERBB4 / | v-erb-a erythro- | NM_005235 | 2066 | 1939 | 600543 |
| HER-4 | blastic leukemia viral oncogene homolog 4 | | | | |
| GRB7 | growth factor receptor-bound protein 7 | NM_0010300 02 | | Hs.868 59 | 601522 |
| MGC9753 | Perl-like domain containing 1PERKD1 | NM_033419 | | Hs.462 971 | |
| THRA | thyroid hormone receptor alpha | NM 199334, NM_003250 | 7067 | Hs.724 | 190120 |
| RARA | retinoic acid receptor alpha | NM_000964 | | Hs.137 731 Hs.654 583 | 180240 |
| TOP02A | topoisomerase (DNA) II alpha 170kDa | NM_001067 | | Hs.156 346 | 126430 |

The terms "Ref. Sequences, Locus_Link_ID, Unigene_ID and OMIM" relate to databases in which the respective proteins are listed under the given access number. These databases can be accessed over the NCBI server.

**Table 2: primer sequences and probe sequences used in accordance with the present invention**

| SEQ ID | Gene | PCR probe | Forward primer | Reverse primer |
|---|---|---|---|---|
| 1-3 | VEGFA | cacattgttggaa gaagcagcccatgac | cagatgtcccggcgaa ga | Gagggcgagtcccaggaa |
| 4-6 | VEGFA | cgttcgtttaact caagctgcctcg | aacacagactcgcgttgcaa | Cggcttgtcacatctgcaagt |
| 7-9 | VEGFA | aacttcctcgggt tcataaccatagcagtcc | cccccaacatctgg ttagtctt | Ccacgggcacagaatatgc |
| 10-12 | VEGFA | caccatgcagattatgcggatcaaacct | gcccactgaggagtccaaca | Tcctatgtgctggcct tggt |
| 13-15 | VEGFA Isoform 121 | caccatgcagattatgcggatcaaacct | gcccactgaggagtccaaca | Gcctcggcttgtcacatttt |
| 16-18 | VEGFA Isoform 165 | caccatgcagattatgcggatcaaacct | gcccactgaggagtccaaca | Agcaaggcccacagggattt |
| 19-21 | VEGFA Isoform 185 | caccatgcagattatgcggatcaaacct | gcccactgaggagtccaaca | aacgctccaggacttataccg |
| 22-24 | VEGFB | acagggctgccac tccccacc | aatgcagacctaaaaaaaaggacagt | Cccagcccggaacagaa |
| 25-27 | VEGFB | cacatctatccatgacaccactttcctctgg | tggcaggtagcgcg agtat | Ccctgtctcccagcct gat |
| 28-30 | VEGFB | ttcctcccctcac taagaagacccaaacct | ccactctgtgcaag taagcatctt | Gtaccaaagcccaaatccca tt |
| 31-33 | VEGFD | tgacattgaaacactaaaagttatagatgaagaatggca | actaggtttgcggcaactttct | Tctctagggctgcact gagttct |
| 34-36 | VEGFC | acggccatgtacg aaccgcca | gttccaccaccaaacatgca | Cactatatgaaaatcctggctcac a |
| 37-39 | VEGFC | aaacatggcccgg cgtcaacc | ccagaatagaagtcatgctttg atg | Tttagatcagagcaaatgtcttgca |
| 40-42 | VEGFC | ttgagtcatctcc agcatccgaggaaa | ccacagatgtcatggaatc cat | Tgcctggctcaggaagattt |
| 43-45 | VEGFC | agaacaggccaac ctcaactcaaggacag | gagatccccatggagg tcttc | Cactcattatcaatacttttcaagatctctgt |
| 46-48 | VEGFC | tgcatgccacggg aggtgtgtataga | aatagaccctggagtgaa accatt | Tattgcagcaacccccacat |
| 49-51 | VEGFC | acatgcagctgtt acagacggccatgt | ctgagcaagcggtctctga gt | Cactatatgaaaatcctggctcac a |
| 52-54 | VEGF-D | ccatcctctaccagaacatacatcagttatttggag a | cccttcccaccaagtgttca | Tggtgctgcctcactg gat |
| 55-57 | ERBB2 | aggccaagtccgc agaagccct | tctggacgtgccag tgtgaa | cctgctccctgaggacacat |
| 58-60 | ERBB2 | accaggacccaccagagcggg | ccagccttcgacaa cctctatt | tgccgtaggtgtccct ttg |
| 61-63 | ERBB2 | tgatcatggtcaaatgt tggatgattgactc | ccatctgcaccattgatgtctac | cggaatcttggccgac att |
| 64-66 | ERBB2 | aagattccccttctt cctggga | acgccctcagaagattggaa | tgtgctgacgcaagctacaac |

### References

1. Shepherd FA, N Engl J Med 2005;353(2):123-32
2. Pao W, J Clin Oncol 2005;23(11):2556-68
3. Tokumo M, Lung Cancer 2006;53(1):117-21
4. Giaccone G, Clin Cancer Res 2006;12(20 Pt 1): 6049-55
5. Oken, MM, Am J Clin Oncol 5:649-655, 1982
6. Konecny GE, Clin Cancer Res. 2004 Dec 15;10(24):8752-3
7. Konecny GE, Clin Cancer Res. 2004 March 1; 10:1706-16
8. Pegram M, Poster Discussion Session III of the 29. San Antonio Breast Cancer Symposium (SABCS), December 14 2006
9. Press MF, Clin Cancer Res. 2005 15; 6598-607.

## Claims

1. A method for predicting a clinical response of a patient suffering from or at risk of developing a neoplastic disease towards a given mode of treatment, said method comprising the steps of:
a) obtaining a biological sample from said patient;
b) determining, on a non protein basis, the expression level of at least one gene encoding for a ligand from the Vascular endothelial growth factor (VEGF) family and of at least one gene encoding for a receptor from the ErbB-family, or a gene co-expressed therewith, in said sample,
c) comparing the pattern of expression levels determined in (b) with one or several reference pattern(s) of expression levels; and
d) predicting therapeutic success for said given mode of treatment in said patient or implementing therapeutic regimen targeting the signalling pathway of said ligand and/or receptor is related to in said patient from the outcome of the comparison in step (c).

2. The method according to claim 1, **characterized in that** the mode of treatment for which prediction is sought is a treatment related to the signalling pathway of a ligand from the Vascular endothelial growth factor (VEGF) family and a treatment related to the signalling pathway of a receptor from the ErbB-family.

3. The method according to any one of the aforementioned claims, **characterized in that** at least one of the said ligand genes the expression level of which is determined is VEGF-A and/or at least one of the receptor genes the expression level of which is determined is Her-2/neu.

4. The method according to any one of the aforementioned claims, said method comprising the additional step of:
e) determining the expression level of a gene encoding for a Growth factor Receptor-Bound Protein (GRB).

5. The method according to any one of the aforementioned claims, wherein upregulated expression of at least one ligand and/or receptor determined in step (b) is indicative of a promising prediction as regards therapeutic success for a mode of treatment or therapeutic regimen related to the signalling pathway of a ligand from the Vascular endothelial growth factor (VEGF) family and/or of a receptor from the ErbB-family.

6. The method according to any one of the aforementioned claims, wherein said given mode of treatment (a) acts on recruitment of lymphatic vessels, angiogenesis, cell proliferation, cell survival and/or cell motility, and/or b) comprises administration of a chemotherapeutic agent.

7. The method according to any one of the aforementioned claims, wherein said given mode of treatment comprises, in addition, chemotherapy, administration of small molecule inhibitors, antibody based regimen, anti-proliferation regimen, pro-apoptotic regimen, pro-differentiation regimen, radiation and/or surgical therapy.

8. The method according to any one of the aforementioned claims, **characterized in that** said given mode of treatment or therapeutic regimen related to the signalling pathway of said ligand and/or receptor comprises adminsitration of at least one agent selected from the group consisting of:
• an agonist of said ligand
• an agonist of a ligand specific for said receptor
• an antibody or an antibody fragment against said ligand and/or receptor,
• an antisense nucleic acid inhibiting the expression of a gene encoding for a said ligand and/or receptor,
• a small molecular drug,
• a kinase inhibitor specific for the given receptor,
• spefifically binding proteins, and/or
• phages.

9. A method of selecting a therapy modality for a patient afflicted with a neoplastic disease, said method comprising the steps of:
a) obtaining a biological sample from said patient;
b) predicting from said sample, by the method according to any one of claims 1 to 8, therapeutic success for a plurality of individual modes of treatment; and
c) selecting a mode of treatment which is predicted to be successful in step (b).

10. The method according to claim 9, comprising
a) obtaining a sample comprising cancer cells from said patient;
b) separately maintaining aliquots of the sample in the presence of one or more test compositions;
c) comparing expression of a single or plurality of molecules, selected from the ligands and/or receptors listed in Table 1 in each of the aliquots; and
d) selecting a test composition which induces a lower level of expression of ligands and/or receptors from Table 1 and/or a higher level of expression of ligands and/or receptors from Table 1 in the aliquot containing that test composition, relative to the level of expression of each ligand in the aliquots containing the other test compositions.

11. The method according to any one of the aforementioned claims, wherein the expression level is determined by
a) a hybridization based method;
b) a PCR based method;
c) a method based on the electrochemical detection of particular molecules, and/or by
d) an array based method.

12. The method according to any one of the aforementioned claims, wherein said cancer or neoplastic disease is selected from the group consisting of gynaecological cancers including Breast cancer, Ovarian cancer, Cervical cancer, Endometrial cancer, Vulval cancer, and the like.

13. The method according to any one of the aforementioned claims, **characterized in that** the expression level of at least one of the said ligands and/or receptors is determined with rtPCR (reverse transcriptase polymerase chain reaction) of the ligand and/or receptor related mRNA.

14. The method according to any one of the aforementioned claims, **characterized in that** the expression level of at least one of the said ligands and/or receptors is determined in fixed and/or paraffin embedded tissue samples.

15. The method according to any one of the aforementioned claims, wherein, after lysis, the samples are treated with silica-coated magnetic particles and a chaotropic salt, in order to purify the nucleic acids contained in said sample for further determination.

16. A kit useful for carrying out a method according to any one of the aforementioned claims, comprising at least
a) a primer pair and/or a probe each having a sequence sufficiently complementary to a gene encoding for a ligand from the VEGF family and/or a receptor from the ErbB family and/or
b) at least an antibody directed against a ligand from the VEGF family and/or a receptor from the ErbB-family.

17. A method for correlating the clinical outcome of a patient suffering from or at risk of developing a neoplastic disease with the presence or non-presence of a defect in expression of a ligand from the VEGF family and/or a receptor from the ErbB-family, said method comprising the steps of:
a) obtaining a fixed biological sample from said patient;
b) determining the expression level of at least one gene encoding for a ligand from the VEGF family and/or one receptor from the ErbB-family in said patient according to any of the above methods, and
c) correlating the pattern of expression levels determined in (b) with said patient's data, said data being selected from the group consisting of etiopathology data, clinical symptoms, anamnesis data and/or data concerning the therapeutic regimen.

18. A nucleic acid molecule, selected from the group consisting of
a) the nucleic acid molecule presented as SEQ ID NO: 1 - 66
b) a nucleic acid molecule having a length of 4 - 80 nucleotides, preferably 18 - 30 nucleotides, the sequence of which corresponds to the sequence of a single stranded fragment of a gene encoding for a ligand and/or receptor selected from the group consisting of VEGFA, VEGFB, VEGFC, FIGF/VEGFD, EGFR/HER-1, ERBB2/Her-2/neu/HER-2, ERBB3/HER-3, ERBB4/HER-4, MGC9753, GRB7, THRA, RARA, and/or TOP02A
c) a nucleic acid molecule that is a fraction, variant, homologue, derivative, or fragment of the nucleic acid molecule presented as SEQ ID NO: 1 - 66
d) a nucleic acid molecule that is capable of hybridizing to any of the nucleic acid molecules of a) - c) under stringent conditions
e) a nucleic acid molecule that is capable of hybridizing to the complement of any of the nucleic acid molecules of a) - d) under stringent conditions
f) a nucleic acid molecule that is capable of hybridizing to the complement of a nucleic acid molecule of e)
g) a nucleic acid molecule having a sequence identity of at least 95 % with any of the nucleic acid molecules of a) - f)
h) a nucleic acid molecule having a sequence identity of at least 70 % with any of the nucleic acid molecules of a) - f)
i) a complement of any of the nucleic acid molecules of a) - h), or
i) a nucleic acid molecule that comprises any nucleic acid molecule of a) - i).

19. The nucleic acid according to claim 18, **characterized in that** the said nucleic acid is selected from the group consisting of DNA, RNA, PNA, LNA and/or Morpholino.

20. The nucleic acid according to any of claims 18 - 19, **characterized in that** it is labelled with at least one detectable marker.

21. A kit of primers and/or detection probes, comprising at least one of the nucleic acids according to any of claims 18 - 20 and/or their fractions, variants, homologues, derivatives, fragments, complements, hybridizing counterparts, or molecules sharing a sequence identity of at least 70%, preferably 95 %.

22. The kit according to claim 21, comprising at least one of the nucleic acid molecules presented as SEQ ID NO: 1 - 66 and/or their fractions, variants, homologues, derivatives, fragments, complements, hybridizing counterparts, or molecules sharing a sequence identity of at least 70%, preferably 95 %, for the detection of at least one gene encoding for a ligand from the VEGF family and/or at least one gene encoding for a receptor from the ErbB-family.

23. Use of a nucleic acid according to any of claims 18 - 20 or of a kit according to any of claims 21 - 22 for predicting a clinical response of a patient suffering from or at risk of developing a neoplastic disease towards a given mode of treatment.
